# EUROPEAN PATENT SPECIFICATION

(11) **EP 3 041 951 B1**
(45) Date of publication and mention of the grant of the patent: **28.11.2018**
(21) Application number: 14765989.0
(22) Date of filing: 04.09.2014
(51) Int. Cl.: C12Q 1/6876, C12Q 1/6883

(54) **CHROMOSOME CONFORMATION CAPTURE METHOD INCLUDING SELECTION AND ENRICHMENT STEPS**
CHROMOSOMENKONFORMATIONSERFASSUNGSVERFAHREN MIT AUSWAHL- UND ANREICHERUNGSSCHRITTEN
PROCÉDÉ DE CAPTURE DE LA CONFORMATION D'UN CHROMOSOME COMPRENANT DES ÉTAPES DE SÉLECTION ET D'ENRICHISSEMENT

(30) Priority: 05.09.2013 GB 201315777
(43) Date of publication of application: 13.07.2016
(73) Proprietor: Babraham Institute, Cambridge, Cambridgeshire CB22 3AT (GB)
(72) Inventor: FRASER, Peter, Cambridge Cambridgeshire CB22 3AT (GB); OSBORNE, Cameron, London SE1 9RT (GB); SCHOENFELDER, Stefan, Cambridge Cambridgeshire CB22 3AT (GB)
(74) Representative: Gibson, Mark
(86) International application number: PCT/GB2014/052664
(87) International publication number: WO 2015/033134

(56) References cited:
- WO-A1-2010/036323
- WO-A1-2012/061600
- WO-A1-2012/108864
- WO-A2-2012/005595
- WO-A2-2012/159025
- JENNIFER L CRUTCHLEY ET AL: "Chromatin conformation signatures: ideal human disease biomarkers?", BIOMARKERS IN MEDICINE, vol. 4, no. 4, 1 August 2010 (2010-08-01), pages 611-629, XP055155789, ISSN: 1752-0363, DOI: 10.2217/bmm.10.68

## Description

### FIELD OF THE INVENTION

The present invention relates to a method for identifying a nucleic acid segment which interacts with a target nucleic acid segment, in particular a method for identifying nucleic acid segments which interact with a sub-group of target nucleic acid segments, as well as kits for performing said method. The invention also relates to a method of identifying one or more interacting nucleic acid segments that are indicative of a particular disease state.

### BACKGROUND OF THE INVENTION

Regulatory elements play a central role in an organism's genetic control and have been shown to contribute to health and disease (e.g. in cancer and autoimmune disorders). Identification of these regulatory elements can also have potential applications in molecular biology, for example, in expression systems and genetic modification techniques. However, while thousands of regulatory elements have been mapped in the mouse and human genomes, determining which target genes they regulate represents a major challenge. It has been demonstrated that regulatory DNA sequences (for example, enhancers) can bridge considerable genomic distances to interact with their target genes. The chromosomal contacts between genes and their regulatory regions can be captured, but the methodology to link genes to their regulatory sequences on a genome-wide scale is currently missing.

One of the first methods developed to identify interactions between genomic loci was Chromosome Conformation Capture (3C) technology (Dekker et al. Science (2002) 295:1306-1311). This involved creating a 3C library by: crosslinking a nuclear composition so that genomic loci that are in close spatial proximity become linked; removing the intervening DNA loop between the crosslink by digestion; and ligating and reverse crosslinking the interacting regions to generate a 3C library. The library can then be used to calculate the frequency of interaction between known sequences. However, this method has a requirement of previous knowledge of the interaction in order to detect the interacting regions of interest.

Since then, the technology has been further developed to try and overcome limitations with the 3C method. For example, the 4C method uses inverse PCR to identify interactions of a bait sequence where the specific interactions are not known (Simonis et al. Nat. Genet. (2006) 38:1341-1347; and Zhao et al. Nat. Genet. (2006) 38:1348-1354). However, this method is costly on both time and resource, is designed to detect interactions with only one bait genomic locus, and is only semi-quantitative.

The Hi-C method uses junction markers to isolate all of the ligated interacting sequences in the cell (see WO 2010/036323 and Lieberman-Aiden et al., Science (2009) 326:289-93). Although this provides information on all the interactions occurring within the nuclear composition at a particular time point, there are around 800,000 ligatable fragments per cell (for example, if a restriction enzyme with a 6 base pair recognition site, such as HindIII or BglII has been used for the Hi-C library generation) which makes the libraries overly-complex and prohibits the interrogation of interactions between specific individual regions.

There is therefore a need to provide an improved method for identifying nucleic acid interactions which overcomes the limitations of the currently available methodologies.

WO 2012/159025 discloses compositions, methods and kits for the analysis of three dimensional chromatin and chromosome conformation using an alternative 3C method and for the diagnosis of disease.

WO 2010/036323 discloses a Hi-C protocol for the identification of spatial co-localisation between genomic loci, including long-range interactions.

WO 2012/005595 discloses a method of determining the sequence of a genomic region of interest comprising use of a target nucleotide sequence and determining the sequences of ligated DNA fragments containing a target nucleotide sequence.

WO 2012/108864 discloses methods for the selective enrichment of nucleic acids comprising a target and utilizing a capture probe contacted to a portion of the target to separate hybridized nucleic acid from nucleic acid not bound by the capture probe.

### SUMMARY OF THE INVENTION

According to a first aspect of the invention, there is provided a method for identifying nucleic acid segments which interact with a sub-group of target nucleic acid segments, said method comprising the steps of:
crosslinking a nucleic acid composition comprising the sub-group of target nucleic acid segments;
fragmenting the crosslinked nucleic acid composition;
marking the ends of the fragments with biotin;
ligating the fragmented nucleic acid segments to produce ligated fragments;
reversing the crosslinking;
shearing the fragments followed by end-repair;
pulldown of fragments with streptavidin;
adapter ligation followed by PCR;
promoter capture by addition of isolating nucleic acid molecules which bind to the sub-group of target nucleic acid segments, wherein said isolating nucleic acid molecules are labelled with a first half of a binding pair;
isolating ligated fragments which contain the sub-group of target nucleic acid segments bound to the isolating nucleic acid molecules by using the second half of the binding pair;
amplification using PCR; and
sequencing to identify the nucleic acid segments which interact with the sub-group of target nucleic acid segments.

According to a further aspect of the invention, there is provided a method of identifying one or more interacting nucleic acid segments that are indicative of a particular disease state comprising:
a) performing the method defined herein on a nucleic acid composition obtained from an individual with a particular disease state;
b) quantifying a frequency of interaction between a nucleic acid segment and a target nucleic acid segment;
c) comparing the frequency of interaction in the nucleic acid composition from the individual with said disease state with the frequency of interaction in a normal control nucleic acid composition from a healthy subject, such that a difference in the frequency of interaction in the nucleic acid composition is indicative of a particular disease state.

According to a further aspect of the invention, there is provided use of a kit for identifying nucleic acid segments which interact with a sub-group of target nucleic acid segments, which comprises buffers and reagents capable of performing the methods defined herein.

### BRIEF DESCRIPTION OF THE FIGURES

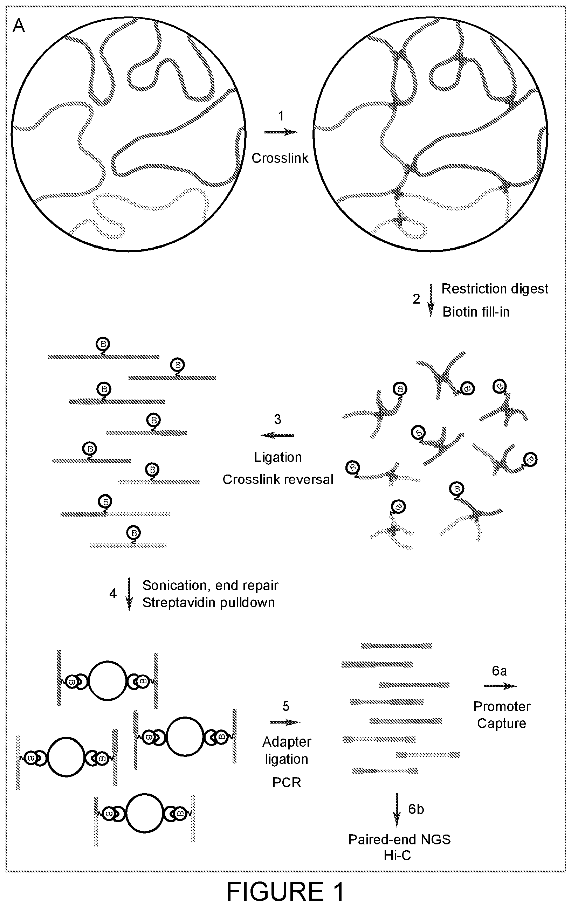

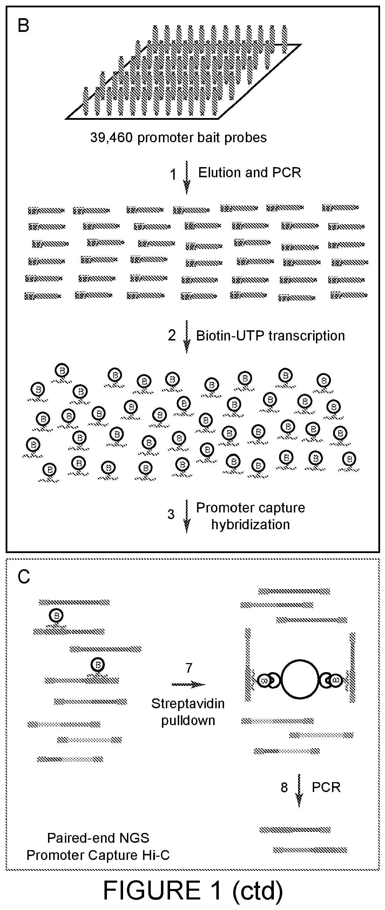

**FIGURE 1****:** A schematic overview of an embodiment of the invention described herein.
**FIGURE 2****:** Results obtained using the method described herein. Figure 2(A) displays the genomic region surrounding the MYC gene and the chromosomal interaction profile of the MYC gene in CD34+ cells. Figure 2(B) shows an example of a DNA FISH of a CD34+ cell nucleus. Figure 2(C) shows validation of the interaction between the MYC promoter and the region at 1.8 Mb downstream of the gene using the 3C method.

### DETAILED DESCRIPTION OF THE INVENTION

According to a first aspect of the invention, there is provided a method for identifying nucleic acid segments which interact with a sub-group of target nucleic acid segments, said method comprising the steps of:
crosslinking a nucleic acid composition comprising the sub-group of target nucleic acid segments;
fragmenting the crosslinked nucleic acid composition;
marking the ends of the fragments with biotin;
ligating the fragmented nucleic acid segments to produce ligated fragments;
reversing the crosslinking;
shearing the fragments followed by end-repair;
pulldown of fragments with streptavidin;
adapter ligation followed by PCR;
promoter capture by addition of isolating nucleic acid molecules which bind to the sub-group of target nucleic acid segments, wherein said isolating nucleic acid molecules are labelled with a first half of a binding pair;
isolating ligated fragments which contain the sub-group of target nucleic acid segments bound to the isolating nucleic acid molecules by using the second half of the binding pair;
amplification using PCR; and
sequencing to identify the nucleic acid segments which interact with the sub-group of target nucleic acid segments.

The method of the present invention provides a means for identifying interacting sequences by using an isolating nucleic acid molecule to isolate a target nucleic acid segment, in particular isolating nucleic acid molecules which isolate a sub-group of target nucleic acid segments. This helps to focus the data on particular interactions within enormously complex libraries and can be used to organize the information into various subsets depending on the type of isolating nucleic acid molecules used (e.g. promoters to identify promoter interactions). Detailed information on the chromosomal interactions within a particular subset can then be obtained.

Whereas previous techniques, such as 4C, allow the capture of genome-wide interactions of one or a few promoters, the method described herein can capture over 22,000 promoters and their interacting genomic loci in a single experiment. Moreover, the present method yields a significantly more quantitative readout.

The Genome-Wide Association Studies (GWAS) have identified thousands of single-nucleotide polymorphisms (SNPs) that are linked to disease. However, many of these SNPs are located at great distances from genes, making it very challenging to predict on which genes they act. Therefore the present method provides a way of identifying interacting nucleic acid segments, even if they are located far away from each other within the genome.

References to "nucleic acid segments" as used herein, are equivalent to references to "nucleic acid sequences", and refer to any polymer of nucleotides (*i.e.,* for example, adenine (A), thymidine (T), cytosine (C), guanosine (G), and/or uracil (U)). This polymer may or may not result in a functional genomic fragment or gene. A combination of nucleic acid sequences may ultimately comprise a chromosome. A nucleic acid sequence comprising deoxyribonucleosides is referred to as deoxyribonucleic acid (DNA). A nucleic acid sequence comprising ribonucleosides is referred to as ribonucleic acid (RNA). RNA can be further characterized into several types, such as protein-coding RNA, messenger RNA (mRNA), transfer RNA (tRNA), long non-coding RNA (InRNA), long intergenic non-coding RNA (lincRNA), antisense RNA (asRNA), micro RNA (miRNA), short interfering RNA (siRNA), small nuclear (snRNA) and small nucleolar RNA (snoRNA).

"Single nucleotide polymorphisms" or "SNPs" are single nucleotide variations (*i.e.* A, C, G or T) within a genome that differ between members of a biological species or between paired chromosomes.

It will be understood that the "target nucleic acid segment" refers to the sequence of interest which is known to the user. Isolating only the ligated fragments which contain the target nucleic acid segment helps to focus the data to identify specific interactions with a particular gene of interest.

References herein to the term "interacts" or "interacting", refer to an association between two elements, for example in the present method, a genomic interaction between a nucleic acid segment and a target nucleic acid segment. The interaction usually causes one interacting element to have an effect upon the other, for example, silencing or activating the element it binds to. The interaction may occur between two nucleic acid segments that are located close together or far apart on the linear genome sequence.

References herein to the term "nucleic acid composition", refers to any composition comprising nucleic acids and protein. The nucleic acids within the nucleic acid composition may be organized into chromosomes, wherein the proteins (*i.e.,* for example, histones) may become associated with the chromosomes having a regulatory function. In one embodiment, the nucleic acid composition comprises a nuclear composition. Such a nuclear composition may typically include a nuclear genome organisation or chromatin.

References to "crosslinking" or "crosslink" as used herein, refers to any stable chemical association between two compounds, such that they may be further processed as a unit. Such stability may be based upon covalent and/or noncovalent bonding (*e.g*. ionic). For example, nucleic acids and/or proteins may be crosslinked by chemical agents (*i.e.,* for example, a fixative), heat, pressure, change in pH, or radiation, such that they maintain their spatial relationships during routine laboratory procedures (*i.e.,* for example, extracting, washing, centrifugation etc.). Crosslinking as used herein is equivalent to the terms "fixing" or "fixation", which applies to any method or process that immobilizes any and all cellular processes. A crosslinked/fixed cell, therefore, accurately maintains the spatial relationships between components within the nucleic acid composition at the time of fixation. Many chemicals are capable of providing fixation, including but not limited to, formaldehyde, formalin, or glutaraldehyde.

References to the term "fragments" as used herein, refers to any nucleic acid sequence that is shorter than the sequence from which it is derived. Fragments can be of any size, ranging from several megabases and/or kilobases to only a few nucleotides long. Fragments are suitably greater than 5 nucleotide bases in length, for example 10, 15, 20, 25, 30, 40, 50, 100, 250, 500, 750, 1000, 2000, 5000 or 10000 nucleotide bases in length. Fragments may be even longer, for example 1, 5, 10, 20, 25, 50, 75, 100, 200, 300, 400 or 500 nucleotide kilobases in length. Methods such as restriction enzyme digestion, sonication, acid incubation, base incubation, microfluidization *etc.,* can all be used to fragment a nucleic acid composition in the second aspect of the invention.

References herein to the term "ligated", refers to any linkage of two nucleic acid segments usually comprising a phosphodiester bond. The linkage is normally facilitated by the presence of a catalytic enzyme (*i.e.,* for example, a ligase such as T4 DNA ligase) in the presence of co-factor reagents and an energy source (*i.e.,* for example, adenosine triphosphate (ATP)). In the method described herein, the fragments of two nucleic acid segments that have been crosslinked are ligated together in order to produce a single ligated fragment.

References herein to an "isolating nucleic acid molecule" refer to a molecule formed of nucleic acids which binds to the target nucleic acid segment. For example, the isolating nucleic acid molecule may contain the complementary sequence to the target nucleic acid segment which will then form interactions with the nucleotide bases of the target nucleic acid segment (*i.e.* to form base pairs (bp)). It will be understood that the isolating nucleic acid molecule, for example biotinylated RNA, does not need to contain the entire complementary sequence of the target nucleic acid segment in order to form complementary interactions and isolate it from the nucleic acid composition. The isolating nucleic acid molecule may be at least 10 nucleotide bases long, for example, at least 20, 30, 40, 50, 60, 70, 80, 90, 100, 130, 150, 170, 200, 300, 400, 500, 750, 1000, 2000, 3000, 4000 or 5000 nucleotide bases long.

References herein to a "binding pair" refer to at least two moieties (*i.e.* a first half and a second half) that specifically recognize each other in order to form an attachment. Suitable binding pairs include, for example, biotin and avidin or biotin and derivatives of avidin such as streptavidin and neutravidin.

References herein to "labelling" or "labelled" refer to the process of distinguishing a target by attaching a marker, wherein the marker comprises a specific moiety having a unique affinity for a ligand (*i.e.* an affinity tag). For example, the label may serve to selectively purify the isolating nucleic acid sequence (*i.e.,* for example, by affinity chromatography). Such a label may include, but is not limited to, a biotin label, a histidine label (*i.e.* 6His), or a FLAG label.

In one embodiment, the target nucleic acid molecule, i.e. the sub-group of target nucleic acid molecules, are selected from a promoter, enhancer, silencer or insulator. In a further embodiment, the target nucleic acid molecule is a promoter. In a further alternative embodiment, the target nucleic acid molecule is an insulator.

References herein to the term "promoter" refer to a nucleic acid sequence which facilitates the initiation of transcription of an operably linked coding region. Promoters are sometimes referred to as "transcription initiation regions". Regulatory elements often interact with promoters in order to activate or inhibit transcription.

The present inventors have used the method of the invention to identify thousands of promoter interactions, with ten to twenty interactions occurring per promoter. The method described herein has identified some interactions to be cell specific, or to be associated with different disease states. A wide range of separation distances between interacting nucleic acid segments has also been identified - most interactions are within 100 kilobases, but some can extend to 2 megabases and beyond. Interestingly, the method has also been used to show that both active and inactive genes form interactions.

Nucleic acid segments that are identified to interact with promoters are candidates to be regulatory elements that are required for proper genetic control. Their disruption may alter transcriptional output and contribute to disease, therefore linking these elements to their target genes could provide potential new drug targets for new therapies.

Identifying which regulatory elements interact with promoters is crucial to understanding genetic interactions. The present method also provides a snapshot look at the interactions within the nucleic acid composition at a particular point in time, therefore it is envisaged that the method could be performed over a series of time points or developmental states or experimental conditions to build a picture of the changes of interactions within the nucleic acid composition of a cell.

It will be understood that in one embodiment the target nucleic acid segment interacts with a nucleic acid segment which comprises a regulatory element. In a further embodiment, the regulatory element comprises an enhancer, silencer or insulator.

The term "regulatory gene" as used herein, refers to any nucleic acid sequence encoding a protein, wherein the protein binds to the same or a different nucleic acid sequence thereby modulating the transcription rate or otherwise affecting the expression level of the same or a different nucleic acid sequence. The term "regulatory element" as used herein, refers to any nucleic acid sequence that affects the activity status of another genomic element. For example, various regulatory elements may include, but are not limited to, enhancers, activators, repressors, insulators, promoters or silencers.

In one embodiment, the target nucleic acid molecule is a genomic site identified through chromatin immunoprecipitation (ChIP) sequencing. ChIP sequencing experiments analyse protein-DNA interactions by crosslinking protein-DNA complexes within a nucleic acid composition. The protein-DNA complex is then isolated (by immunoprecipitation) prior to sequencing the genomic region to which the protein is bound.

It will be envisaged that in some embodiments, the nucleic acid segment is located on the same chromosome as the target nucleic acid segment. Alternatively, the nucleic acid segment is located on a different chromosome to the target nucleic acid segment.

The method may be used to identify a long range interaction, a short range interaction or a close neighbour interaction. The term "long range interaction" as used herein, refers to the detection of interacting nucleic acid segments that are far apart within the linear genome sequence. This type of interaction may identify two genomic regions that are, for instance, located on different arms of the same chromosome, or located on different chromosomes. The term "short range interaction" as used herein, refers to the detection of interacting nucleic acid segments that are located relatively close to each other within the genome. The term "close neighbour interaction" as used herein, refers to the detection of interacting nucleic acid segments that are very close to each other in the linear genome and, for instance, part of the same gene.

SNPs have been shown by the present inventors to be positioned more often in an interacting nucleic acid segment than would be expected by chance, therefore the method of the present disclosure can be used to identify which SNPs interact, and are therefore likely to regulate, specific genes.

In one embodiment, the isolating nucleic acid molecules are obtained from bacterial artificial chromosomes (BACs), fosmids or cosmids. In a further embodiment, the isolating nucleic acid molecules are obtained from bacterial artificial chromosomes (BACs).

In one embodiment, the isolating nucleic acid molecules are DNA, cDNA or RNA. In a further embodiment, the isolating nucleic acid molecules are RNA.

The isolating nucleic acid molecule may be employed in a suitable method, such as solution hybridization selection (see WO2009/099602). In this method a set of 'bait' sequences is generated to form a hybridization mixture that can be used to isolate a sub group of target nucleic acids from a sample (*i.e.* 'pond').

In one embodiment, the first half of the binding pair comprises biotin and the second half of the binding pair comprises streptavidin.

In one embodiment, the fragmenting step is performed using a restriction enzyme or sonication. In a further embodiment, the fragmenting step is performed using a restriction enzyme.
The term "restriction enzyme" as used herein, refers to any protein that cleaves nucleic acid at a specific base pair sequence. Cleavage can result in a blunt or sticky end, depending on the type of restriction enzyme chosen. Examples of restriction enzymes include, but are not limited to, EcoRI, EcoRII, BamHI, HindIII, DpnII, BglII, NcoI, TaqI, NotI, HinfI, Sau3A, PvuII, SmaI, HaeIII, HgaI, AluI, EcoRV, KpnI, PstI, SacI, SalI, ScaI, SpeI, SphI, StuI, XbaI. In one embodiment, the restriction enzyme is HindIII.

In one embodiment, the method additionally comprises incorporating a junction marker into the ligated fragments during the ligating step.

The term "junction marker" as used herein, refers to any compound or chemical moiety that is capable of being incorporated within a nucleic acid and can provide a basis for selective purification. For example, a junction marker may include, but not be limited to, a labeled nucleotide linker (*e.g*. biotin), a labeled and/or modified nucleotide, nick translation, primer linkers, or tagged linkers.

The junction marker allows ligated fragments to be purified prior to the isolating step, therefore this ensures that only ligated sequences are bound by the isolating nucleic acid molecule, rather than non-ligated (*i.e.* non-interacting) fragments.

In one embodiment, the junction marker comprises a labeled nucleotide linker (*i.e.,* for example, biotin). In a further embodiment, the junction marker comprises biotin. In one embodiment, the junction marker comprises a modified nucleotide. In one embodiment, the junction marker comprises a primer linker.

In one embodiment, the nucleic acid fragments are prevented from ligating to each other in the ligating step, without the addition of a junction marker, *i.e.* the junction marker will contain a linker sequence.

It will be understood that there are several ways known in the art to reverse crosslinks and it will depend upon the way in which the crosslinks are originally formed. For example, crosslinks may be reversed by subjecting the crosslinked nucleic acid composition to high heat, such as above 50°C, 55°C, 60°C, 65°C, 70°C, 75°C, 80°C, 85 °C, or greater. Furthermore, the crosslinked nucleic acid composition may need to be subjected to high heat for longer than 1 hour, for example, at least 5 hours, 6 hours, 7 hours, 8 hours, 9 hours, 10 hours or 12 hours or longer. In one embodiment, reversing the cross-linking comprises incubating the crosslinked nucleic acid composition at 65°C for at least 8 hours (*i.e.* overnight) in the presence of Proteinase K.

In one embodiment, the method additionally comprises purifying the nucleic acid composition to remove any fragments which do not contain the junction marker prior to the promoter capture step.

References herein to "purifying", may refer to a nucleic acid composition that has been subjected to treatment (*i.e.,* for example, fractionation) to remove various other components, and which composition substantially retains its expressed biological activity. Where the term "substantially purified" is used, this designation will refer to a composition in which the nucleic acid forms the major component of the composition, such as constituting about 50%, about 60%, about 70%, about 80%, about 90%, about 95% or more of the composition (*i.e.,* for example, weight/weight and/or weight/volume).

In one embodiment, the method additionally comprises ligating paired end adapter sequences to the ends of the isolated target ligated fragments prior to the sequencing step.

The term "paired end adapters" as used herein, refers to any primer pair set that allows automated high throughput sequencing to read from both ends simultaneously. For example, such high throughput sequencing devices that are compatible with these adaptors include, but are not limited to Solexa (Illumina), the 454 System, and/or the ABI SOLiD. For example, the method may include using universal primers in conjunction with poly-A tails.

In one embodiment, the amplification step is performed by polymerase chain reaction (PCR).

In one embodiment, the nucleic acid composition is derived from a mammalian cell nucleus. In a further embodiment, the mammalian cell nucleus may be a human cell nucleus. Many human cells are available in the art for use in the method described herein, for example GM12878 (a human lymphoblastoid cell line) or CD34+ (human *ex vivo* haematopoietic progenitors).

It will be appreciated that the method described herein finds utility in a range of organisms, not just humans. For example, the present method may also be used to identify genomic interactions in plants and animals.

Therefore, in an alternative embodiment, the nucleic acid composition is derived from a non-human cell nucleus. In one embodiment, the non-human cell is selected from the group including, but not limited to, plants, yeast, mice, cows, pigs, horses, dogs, cats, goats, or sheep. In one embodiment, the non-human cell nucleus is a mouse cell nucleus or a plant cell nucleus.

According to a further aspect of the invention, there is provided a method of identifying one or more interacting nucleic acid segments that are indicative of a particular disease state comprising:
a) performing the method defined herein on a nucleic acid composition obtained from an individual with a particular disease state;
b) quantifying a frequency of interaction between a nucleic acid segment and a target nucleic acid segment;
c) comparing the frequency of interaction in the nucleic acid composition from the individual with said disease state with the frequency of interaction in a normal control nucleic acid composition from a healthy subject, such that a difference in the frequency of interaction in the nucleic acid composition is indicative of a particular disease state.

References to "frequency of interaction" or "interaction frequency" as used herein, refers to the number of times a specific interaction occurs within a nucleic acid composition (*i.e.* sample). In some instances, a lower frequency of interaction in the nucleic acid composition, compared to a normal control nucleic acid composition from a healthy subject, is indicative of a particular disease state (*i.e.* because the nucleic acid segments are interacting less frequently). Alternatively, a higher frequency of interaction in the nucleic acid composition, compared to a normal control nucleic acid composition from a healthy subject, is indicative of a particular disease state (*i.e.* because the nucleic acid segments are interacting more frequently). In some instances, the difference will be represented by at least a 0.5-fold difference, such as a 1-fold, 1.5-fold, 2-fold, 2.5-fold, 3-fold, 4-fold, 5-fold, 7-fold or 10-fold difference.

In one aspect of the disclosure, the frequency of interaction may be used to determine the spatial proximity of two different nucleic acid segments. As the interaction frequency increases, the probability increases that the two genomic regions are physically proximal to one another in 3D nuclear space. Conversely, as the interaction frequency decreases, the probability decreases that the two genomic regions are physically proximal to one another in 3D nuclear space.

Quantifying can be performed by any method suitable to calculate the frequency of interaction in a nucleic acid composition from a patient or a purification or extract of a nucleic acid composition sample or a dilution thereof. For example, high throughput sequencing results can also enable examination of the frequency of a particular interaction. In methods of the disclosure, quantifying may be performed by measuring the concentration of the target nucleic acid segment or ligation products in the sample or samples. The nucleic acid composition may be obtained from cells in biological samples that may include cerebrospinal fluid (CSF), whole blood, blood serum, plasma, or an extract or purification therefrom, or dilution thereof. In one embodiment, the biological sample may be cerebrospinal fluid (CSF), whole blood, blood serum or plasma. Biological samples also include tissue homogenates, tissue sections and biopsy specimens from a live subject, or taken post-mortem. The samples can be prepared, for example where appropriate diluted or concentrated, and stored in the usual manner.

In one embodiment, the disease state is selected from: cancer, autoimmune disease, a developmental disorder, a genetic disorder, diabetes, cardiovascular disease, kidney disease, lung disease, liver disease, neurological disease, viral infection or bacterial infection. In a further embodiment, the disease state is cancer or autoimmune disease. In a yet further embodiment, the disease state is cancer, for example breast, bowel, bladder, bone, brain, cervical, colon, endometrial, esophageal, kidney, liver, lung, ovarian, pancreatic, prostate, skin, stomach, testicular, thyroid or uterine cancer, leukemia, lymphoma, myeloma or melanoma.

References herein to an "autoimmune disease" include conditions which arise from an immune response targeted against a person's own body, for example Acute disseminated encephalomyelitis (ADEM), Ankylosing Spondylitis, Behçet's disease, Celiac disease, Crohn's disease, Diabetes mellitus type 1, Graves' disease, Guillain-Barré syndrome (GBS), Psoriasis, Rheumatoid arthritis, Rheumatic fever, Sjögren's syndrome, Ulcerative colitis and Vasculitis.

References herein to a "developmental disorder" include conditions, usually originating from childhood, such as learning disabilities, communication disorders, Autism, Attention-deficit hyperactivity disorder (ADHD) and Developmental coordination disorder.

References herein to a "genetic disorder" include conditions which result from one or more abnormalities in the genome, such as Angelman syndrome, Canavan disease, Charcot-Marie-Tooth disease, Color blindness, Cri du chat syndrome, Cystic fibrosis, Down syndrome, Duchenne muscular dystrophy, Haemochromatosis, Haemophilia, Klinefelter syndrome, Neurofibromatosis, Phenylketonuria, Polycystic kidney disease, Prader-Willi syndrome, Sickle-cell disease, Tay-Sachs disease and Turner syndrome.

According to a further aspect of the invention, there is provided use of a kit for identifying nucleic acid segments which interact with a sub-group of target nucleic acid segments, which comprises buffers and reagents capable of performing the methods defined herein.

According to a further aspect of the disclosure which may be mentioned, there is provided a kit for identifying a nucleic acid segment which interacts with a target nucleic acid segment, which comprises buffers and reagents capable of performing the methods defined herein.

The kit may include one or more articles and/or reagents for performance of the method. For example, an oligonucleotide probe and/or pair of amplification primers for use in the methods described herein may be provided in isolated form and may be part of a kit, *e.g.* in a suitable container such as a vial in which the contents are protected from the external environment. The kit may include instructions for use according to the protocol of the method described herein. A kit wherein the nucleic acid is intended for use in PCR may include one or more other reagents required for the reaction, such as polymerase, nucleotides, buffer solution etc.

It will be understood that examples of the types of buffers and reagents to be included in the kit can be seen in the Examples described herein.

The following studies and protocols illustrate embodiments of the methods described herein:

### Abbreviations:

- BAC: Bacterial Artificial Chromosome
- BB: Binding buffer
- BSA: Bovine Serum Albumin
- dd: dideoxy
- DMEM: Dulbecco's Modified Eagle Medium
- EDTA: Ethylenediaminetetraacetic acid
- FBS: Foetal Bovine Serum
- NaCl: Sodium Chloride
- NTB: No Tween Buffer
- PBS: Phosphate Buffered Saline
- PCR: Polymerase Chain Reaction
- PE: Paired-end
- rpm: Revolutions Per Minute
- SCRiBL: Sequence Capture of Regions interacting with Bait Loci
- SDS: Sodium Dodecyl Sulphate
- SPRI beads: Solid Phase Reversible Immobilisation beads
- TB: Tween buffer
- Tris-HCl: Tris(hydroxymethyl)aminomethane Hydrochloride
- WB: Wash buffer

### EXAMPLE 1: Promoter Capture Hi-C protocol

### Day 1: Fixation of cells, HindIII digestion

1. Start with 3×10⁷ to 4×10⁷ cells. Make up to 37 ml with room temperature DMEM/10% FBS.
2. Add formaldehyde to a final concentration of 2% and fix for exactly 10 minutes at room temperature while mixing on a rocker.
3. Quench reaction by adding 6 ml cold 1M glycine (0.125 M final).
4. Incubate for 5 minutes at room temperature, followed by 15 minutes on ice.
5. Centrifuge at 1500rpm (400xg) for 10 minutes at 4°C.
6. Discard supernatant, re-suspend pellet carefully in cold 1×PBS, add cold 1×PBS to a final volume of 50ml.
7. Centrifuge at 1500rpm (400xg) for 10 minutes at 4°C, then discard supernatant. The cells can be flash frozen in liquid nitrogen and stored cells at - 80°C.
8. Resuspend cells in 50ml ice-cold lysis buffer (10 mM Tris-HCI pH 8, 10 mM NaCl, 0.2% Igepal CA-630, add one tablet protease inhibitor cocktail (Roche complete, EDTA-free, 11873580001). Make fresh just before use.
9. Optional, but recommended for some cell types: dounce homogenize the cells (2 x 10 strokes, with a 5 minute break in between, while cells are kept on ice) during the 30 minutes incubation (see step 10 below).
10. Incubate on ice for 30 minutes with occasional mixing.
11. Spin the chromatin at 1800 rpm for 5 minutes at 4°C, discard supernatant.
12. Carefully layer approximately 500µl 1.25x NEBuffer 2 on top of the cell pellet, but do not resuspend. This step is to remove any remaining lysis buffer. Discard the supernatant.
13. Resuspend the pellet in 250µl of 1.25x NEBuffer 2 per 5 to 6 Mio cells.
   For example: when starting with 30 Mio cells, resuspend pellet in 1.5ml 1.25x NEBuffer 2.
14. Split into 250µl aliquots corresponding to 5 to 6 Mio cells each (starting). For example: when starting with 30 Mio cells, divide in 6 aliquots with 250µl each.
15. To each of the aliquots, add 108 µl 1.25x NEBuffer 2 (final volume 358 µl).
16. Add 11 µl 10% SDS per tube, mix carefully, and incubate at 37°C for 60 minutes, rotating at 950 rpm.
   This step removes proteins that were not directly cross-linked to the DNA.
17. Quench the SDS by adding 75µl 10% Triton X-100 per tube, and incubate at 37°C for 60 minutes, rotating at 950 rpm.
18. Digest the chromatin by adding 1500 units of HindIII (NEB R0104T) per tube and incubate at 37°C overnight while rotating (950 rpm).

### Day 2: Biotinylation of DNA ends, ligation

Note: start this day by preparing ligation buffer mix (see Day 2, step 5), but add 10x ligation buffer only just before use.
1. Place tubes on ice. Perform biotinylation step with Hi-C samples as described below.
2. To fill in the restriction fragment overhangs and mark the DNA ends with biotin, add 6µl 10x NEB2, 2µl H₂O, 1.5µl 10mM dCTP, 1.5µl 10mM dGTP, 1.5µl 10mM dTTP, 37.5µl 0.4mM biotin-14-dATP (Life Technologies 19524-016), and 10µl 5U/µl Klenow (DNA polymerase I large fragment, NEB M0210L). Mix carefully and incubate for 60 minutes at 37°C. Do not rotate but pipette up and down carefully (approximately every 10 minutess) to mix, avoiding bubbles.
3. Place tubes on ice. To inactivate the enzymes, add 86µl 10% SDS (final concentration 1.47%) to all tubes.
4. Incubate enzymes at 65°C for exactly 30 minutes at 950 rpm and place tubes on ice immediately afterwards.
5. During the incubation (Day 2, step 4), prepare one 15 ml falcon tube per sample with each 7.61 ml ligation mix (745µl 10% Triton X-100, 820µl 10x ligation buffer (NEB B0202S), 82µl 10mg/ml BSA (NEB B9001S), and 5.965 ml water). Transfer each digested chromatin mixture (Day 2, step 4) to the corresponding 15 ml tube.
6. Incubate at 37°C for 60 minutes, with occasional mixing
7. Add 50µl 1U/µl T4 DNA ligase (Invitrogen 15224-025) to each tube. Mix by inverting the tubes and incubate for 4 hours at 16°C.
8. Incubate a further 30 minutes at room temperature.
9. Crosslinks are reversed and protein is degraded by adding 60µl 10mg/ml proteinase K (Roche 03115879001) per tube and incubating the tubes overnight at 65°C.

### Day 3: DNA purification part I

1. After the overnight incubation, add an additional 60µl 10 mg/ml proteinase K per tube and continue the incubation at 65°C for another 2 hours.
2. Cool the reaction mixtures to room temperature. Add 12.5µl of 10mg/ml RNase A (Roche 10109142001) and incubate at 37°C for 60 minutes.
3. Purify the DNA in these tubes by performing a phenol extraction. Transfer the reaction mixtures to 50ml falcon tubes, and add 8ml phenol pH8.0 (Sigma P4557). Vortex for 1 minute, and spin the tubes for 10 minutes at 3500 rpm, then carefully transfer as much of the aqueous phase as possible to a new 50 ml falcon tube.
4. 'Back-extraction': transfer 2.5ml of 1xTE (10mM Tris-HCI pH8, 1mM EDTA) to the tubes containing the lower phase from the Day 3, step 3. Vortex for 1 minute, and spin the tubes for 10 minutes at 3500 rpm, then carefully transfer as much of the aqueous upper phase as possible to the supernatant in the corresponding tube from Day 3, step 3.
5. Repeat the extraction using 10ml phenol pH8.0:chloroform (Sigma P3803), following the steps described on Day 3, step 3.
6. To precipitate the DNA, add 1000µl 3M sodium acetate pH5.2, and 25 ml of ice cold 100 % ethanol to each tube. Incubate overnight at -20°C.

### Day 4: DNA purification part II

1. Spin at 3500 rpm at 4°C for 30 minutes.
2. Discard the supernatant and resuspend each pellet in 400µl 1xTE. The DNA can be stored at -20°C at this step if necessary.
3. Another round of purification is performed by doing 2 phenol:chloroform extractions. Add 450µl phenol pH 8.0:chloroform (1:1) and vortex for 1 minute. Centrifuge the tubes for 10 minutes at 14,000 rpm and transfer the aqueous phase to a new tube. After the second extraction, precipitate the DNA by adding 0.1x volume of 3M sodium acetate pH 5.2 and 2.5x volume of 100% ethanol, and incubate overnight at -20 °C.

### Day 5: DNA purification part III

1. After spinning down the precipitated DNA (14,000 rpm at 4°C for 30 minutes), wash each DNA pellet three times with 70% ethanol and re-suspend each DNA pellet in 25µl 1x TE.
2. Pool the contents of all Hi-C tubes.
3. Use the Quant-iT PicoGreen (Life Technologies P7589) assay to determine the DNA yields. Make 1:200, 1:500 and 1:1000 dilutions for each of the Hi-C libraries.

### Day 6 + 7: Hi-C ligation efficiency and quality controls

1. To check the quality and quantity of the libraries, run 2µl and 6µl aliquots of 1:10 dilutions from the Hi-C libraries on a 0.8% agarose gel. The libraries should run as a rather tight band over 10 kb.
2. Hi-C marking and Hi-C ligation efficiency is verified by a PCR digest assay. Successful fill-in and ligation of a HindIII site (AAGCTT) creates a site for the restriction enzyme NheI (GCTAGC).
   Set up five (identical) 25µl PCR reactions to amplify a short-range ligation product (formed from two nearby restriction fragments), for example the Fraser lab *Calr* control. Use 200 ng of each Hi-C library as template. The PCR products are then pooled, purified (Qiagen PCR purification kit), and the concentration is determined (Nanodrop). Subsequently, split into four samples (undigested, digest with HindIII, digest with NheI, and digest with both HindIII and NheI). Aim for 500 to 600 ng of PCR product per digest. After running the digested samples on a 1.5% gel, the relative number of 3C and Hi-C ligation events can be estimated by quantifying the intensity of the cut and uncut bands.
3. Check for known long-range interactions, such as between *Hbb* and *Eraf* in mouse erythroid cells, in the Hi-C libraries.

### Day 8: Removal of biotin from non-ligated DNA ends

1. Biotin-14-dATP at non-ligated DNA ends is removed with the exonuclease activity of T4 DNA polymerase. For each reaction (set up at least 8 reactions in total per Hi-C library, corresponding to 40µg total), mix 5µg of Hi-C library with 0.5µl 10 mg/ml BSA, 5µl 10x NEBuffer 2, 2µl 2.5mM dATP, and 5µl T4 DNA polymerase (NEB M0203L) in a total volume of 50µl. Incubate at 20°C for 4 hours.
2. Stop the reaction by adding 2ml 0.5 M EDTA pH 8.0 to each tube.
3. Pool 2 reactions each from Day 8, step 1 (total amount of DNA now approximately 10µg per sample).
   To purify the DNA, phenol:chloroform extract, followed by ethanol precipitation as described on Day 4, step 3. Incubate for several hours to overnight at -20°C.
4. Spin samples for 30 minutes at 14,000 rpm at 4°C. Discard supernatant and wash once with 70% ethanol. Discard supernatant, air-dry DNA pellets and resuspend in 130µl H₂O per sample (each sample should contain around 10mg of DNA at this stage).

### Day 9: DNA shearing and end repair

1. Use Covaris E220 for sonication. Settings for DNA shearing to obtain fragments with a peak around 400 bp:
   Duty Factor 10%
   Peak Incident Power (w): 140
   Cycles per burst: 200
   Time: 55 seconds
2. After sonication, transfer the entire volume of each sample (130µl) into a fresh Eppendorf tube. Add the following:
   18µl 10x ligation buffer (NEB B0202S)
   18µl 2.5mM dNTP mix
   6.5µl T4 DNA polymerase (NEB M0203L)
   6.5µl T4 DNA Polynucleotide kinase (NEB M0201L)
   1.3µl Klenow (NEB M0210L)
   Incubate at room temperature for 30 minutes.
3. Split each sample into two (each containing around 5µg DNA, which is the maximal capacity for the columns used below).
   Purify DNA with MinElute columns (Qiagen 28004) according to the manufacturer's instructions. Elute each column with 15µl TLE (10mM Tris pH8.0, 0.1mM EDTA), and repeat the elution with another 15µl TLE.

### Day 10: Addition of dATP, SPRI bead size selection

1. To the sheared and end repaired DNA from Day 9, step 3 (30µl), add the following:
   5µl of NEBuffer 2 10x
   11.5µl dATP 1mM
   3.5µl Klenow exo- (NEB M0212L)
   Incubate at 37°C for 30 minutes.
2. To inactivate the enzyme, incubate at 65°C for 20 minutes, put on ice immediately afterwards.
3. Size select fragments between 200 and 650 base pairs by double-sided SPRI bead size selection (0.6x followed by 0.9x). Mix SPRI beads (Beckman Coulter Ampure XP beads A63881) well by vortexing, keep at room temperature for at least 30 minutes.
   a) Pool two A-tailed samples from Day 10, step 1 (total volume now 100µl) into a fresh tube (A).
   b) For each sample, prepare one tube (B) with 180µl SPRI bead solution.
   c) Add 60µl of SPRI bead solution out of tube (B) into tube (A) with the 100µl of DNA solution (0.6x). Mix thoroughly, incubate for 10 minutes at room temperature, and place on magnetic separator and recover the unbound supernatant containing the DNA in the desired size range into a fresh tube (tube C). Discard tube (A) containing the beads.
   d) Concentrate the SPRI beads: Place tube (B) (containing 120µl of SPRI beads in solution) on the magnet, and remove all but 30µl of the supernatant (*i.e.* discard around 90µl of the supernatant). Take tube (B) off the magnet and resuspend the beads in the remaining 30µl volume.
   e) Add 30µl of concentrated beads (see above) from tube (B) into tube (C) (0.9x SPRI bead, *i.e.* ratio of DNA to SPRI beads in solution is now 1:0.9). Mix well, incubate at least 10 minutes at room temperature, place on magnet, and discard supernatant. Wash twice with freshly prepared 70% ethanol. Discard tube (B).
4. Resuspend bead-bound DNA in tube (C) in 50µl TLE (Tris low-EDTA; 10mM Tris, 0.1mM EDTA), incubate at room temperature for 5 minutes, place on magnet and transfer supernatant (containing your size-selected DNA) into a fresh tube (D). Discard tube (C) containing the beads.
5. Pool all Hi-C library samples.

### Day 11: Biotin-streptavidin pulldown and adapter ligation

1. Use the Quant-iT PicoGreen (Life Technologies P7589) assay to determine the DNA yields. Prepare 1:20 and 1:50 dilutions for your sample(s). The expected yield from 40µg starting material is about 10µg.
   To avoid overloading the beads (see Day 11, step 4), it is recommended to not use more than a maximum of 2.5µg of DNA per 150µl of streptavidin beads.
2. Generate PE adapters by annealing PE adapter primers 1 and 2:
   5'-P-GATCGGAAGAGCGGTTCAGCAGGAATGCCGAG-3' (SEQ ID NO: 1)
   5'-ACACTCTTTCCCTACACGACGCTCTTCCGATCT-3' (SEQ ID NO: 2)

   Mix 15µl of each PE adapter primer with 70µl H₂O (total volume 100µl) . In a PCR machine, run the following program: 95°C for 5 minutes, then decrease in temperature by 1°C per minute, until 4°C is reached.
   Make PE adapter aliquots (15µM) of 10 to 20µl, store at -20°C, and thaw aliquot just before use.
3. Prepare buffers:
   a) TB (Tween buffer): 5mM Tris, 0.5mM EDTA, 1M NaCl, 0.05% Tween
      Prepare 1600µl per sample
   b) NTB (No Tween buffer): 5mM Tris, 0.5mM EDTA, 1M NaCl
      Prepare 600µl per sample
   c) NTB 2x: 10mM Tris, 1mM EDTA, 2M NaCl
      Prepare 300µl per sample
   d) Ligation buffer (10x ligation buffer NEB B0202S): 1x
      Prepare 150µl per sample
   e) NEBuffer 2: 1x
      Prepare 300µl per sample
4. Transfer 150µl of Dynabeads MyOne Streptavidin C1 beads (Life Technologies 650.01) suspension into a lowbind 1.5ml Eppendorf tube.
   Set up one reaction per 2µg to 2.5µg of DNA as determined on Day 11, step 1.
5. Wash beads twice with 400µl TB. Wash steps are always:
   a) Place sample on magnetic separator and reclaim beads
   b) Discard supernatant
   c) Add new buffer, mix thoroughly, and transfer sample to a new tube
   d) Rotate sample for 3 minutes at room temperature
   e) see a)
6. Resupend beads in 300µl of NTB 2x. Make up volume of Hi-C DNA (see Day 11, step 1) to 300µl with TLE.
   In case the amount of Hi-C library DNA exceeds 2.5µg (see Say 11, steps 1 and 4), prepare the adequate number of Hi-C samples, each containing a maximum of 2.5µg of DNA in a total volume of 300µl TLE.
   Combine the beads with the Hi-C DNA (total volume 600µl). Rotate slowly for 30 minutes at room temperature.
7. Place sample on magnetic separator, reclaim beads with bound Hi-C DNA, discard supernatant and wash once with 400µl NTB, followed by another wash with 200µl of 1x ligation buffer.
8. Resuspend in 50µl 1x ligation buffer and transfer to a fresh tube. Add 4µl of annealed 15µM PE adapter (see Day 11, step 2), and 4µl of T4 DNA ligase (NEB M0202S). Rotate slowly at room temperature for 2 hours.
9. Place sample on magnetic separator, reclaim Hi-C bound beads, and wash twice with 400µl TB each.
10. Wash with 200µl 1xNTB, then wash with 200µl 1xNEBuffer 2.
11. Wash with 60µl 1xNEBuffer 2, then resuspend beads in 40µl 1xNEBuffer 2 and transfer into a fresh tube. If more than one streptavidin-biotin pulldown per Hi-C library was performed (*i.e.* if the starting amount of Hi-C library exceeded 2.5µg DNA, see Day 11, steps 1 and 4), pool all reactions. Store at 4°C.

### Day 12: Test PCRs to determine conditions for Hi-C library amplification

1. To determine the optimal number of PCR cycles for Hi-C library, set up test PCRs with 6, 9, 12, and 15 amplification cycles. Set up 4 reactions, each with:

| | |
|---|---|
| 2.5µl | Hi-C library DNA on beads |
| 5µl | Buffer 5x (Phusion NEB F531) |
| 0.7µl | dATP 10mM |
| 0.7µl | dCTP 10mM |
| 0.7µl | dGTP 10mM |
| 0.7µl | dTTP 10mM |
| 0.075µl | PE PCR primer 1.0 (100µM) |
| 0.075µl | PE PCR primer 2.0 (100µM) |
| 0.3µl | Phusion polymerase (NEB F531) |
| 14.25µl | H₂O |

PE PCR Primer 1.0:
   AATGATACGGCGACCACCGAGATCTACACTCTTTCCCTACACGACGCTCTTCCGATCT (SEQ ID NO: 3)
PE PCR Primer 2.0:

2. Run 4 different PCRs with following conditions for n cycles:

| | | |
|---|---|---|
| 1 cycle: | 98°C | 30 seconds |
| | 65°C | 30 seconds |
| | 72°C | 30 seconds |
| n - 2 cycles: | 98°C | 10 seconds |
| | 65°C | 30 seconds |
| | 72°C | 30 seconds |
| 1 cycle: | 98°C | 10 seconds |
| | 65°C | 30 seconds |
| | 72°C | 7 minutes |

3. Check the amount of amplified DNA by running the entire reaction (25µl) on a 1.5% agarose gel. Usually, a smear in the range of 300 to 600 bp should be just about visible at 9 (in some cases 6) cycles of amplification and increase in intensity with increasing number of PCR cycles.

### Day 13: Final PCR amplification of Hi-C library

1. Set up x PCR reactions (remaining volume of HiC library divided by factor 2.5) with 25µl each, as described on Day 12, step 1, with one PCR condition (*i.e.* number of cycles). The number of the PCR cycles will depend on the available amount of Hi-C library, and the test PCRs (Day 12), but typically 6 to 7 cycles should be sufficient to obtain approximately 500 ng of Hi-C library required for capture Hi-C (see below).
2. Pool all individual PCR reactions from Day 13, step 1. Place on a magnetic separator, and transfer the supernatant into a fresh 1.5ml lowbind Eppendorf tube. Resuspend the streptavidin beads in the original amount of 1xNEBuffer 2 (see Day 11, step 11) and keep as a backup. Determine the volume of the supernatant containing the amplified Hi-C library, and purify by adding 1.8x volume of SPRI beads (Beckman Coulter Ampure XP beads A63881), following the manufacturer's instructions. Resuspend in 100µl of TLE.
3. Repeat SPRI bead purification (as described on Day 13, step 2), by adding 180µl of SPRI beads to 100µl of Hi-C library from Day 13, step 2. Resuspend in a final volume of 20µl TLE.
4. Check the quality and quantity of the Hi-C library by Bioanalyzer (Agilent). Keep 50ng to 100ng of the Hi-C library back for next generation sequencing.

### Day 14: Hybridization of Hi-C library with biotin-RNA

1. Prepare Hi-C library (pond): transfer volume equivalent to 500ng of Hi-C library into a 1.5 ml Eppendorf tube and concentrate using a SpeedVac. Keep 50ng to 100ng Hi-C library back for next generation sequencing. After evaporation of all liquid, resuspend the Hi-C DNA pellet in 3.4µl of H₂O. Add the following components:
   2.5µl custom blocker 1 (Agilent Technologies)
   2.5µl custom blocker 2 (Agilent Technologies)
   0.6µl custom blocker 3 (Agilent Technologies)
   Resuspend thoroughly, transfer into PCR strip (Agilent 410022), close with a PCR strip tube lid (Agilent optical cap 8x strip 401425) and keep on ice.
2. Prepare hybridization buffer (49µl per Capture Hi-C sample) by mixing the following components:
   25µl SureSelect Hybridization solution 1 (Agilent Technologies)
   1µl SureSelect Hybridization solution 2 (Agilent Technologies)
   10µl SureSelect Hybridization solution 3 (Agilent Technologies)
   13µl SureSelect Hybridization solution 4 (Agilent Technologies)

   Mix thoroughly, heat to 65°C for 5 minutes, transfer into PCR strip (Agilent 410022), close with a PCR strip tube lid (Agilent optical cap 8x strip 401425) and keep at room temperature.
3. Prepare biotin-RNA (bait): transfer 5µl biotinylated RNA (100ng/µl, custom made, Agilent Technologies) into a 1.5ml lowbind Eppendorf tube.
   Use nuclease-free water to prepare a 1:4 dilution of SureSelect RNase Block (Agilent Technologies), and add 2µl of the RNase Block dilution to the biotinylated RNA. Mix well, transfer into a PCR strip (Agilent 410022), close with a PCR strip tube lid (Agilent optical cap 8x strip 401425) and keep on ice.
4. Hybridization reaction: Set the PCR machine (PTC-200, MJ Research) to the following program:
   1. 95°C for 5 minutes
   2. 65°C forever

The PCR machine lid has to be heated. Throughout the procedure, work quickly and try to keep the PCR machine lid open for the minimum time possible. Evaporation of the sample will results in suboptimal hybridization conditions.

Transfer the PCR strip with the pond Hi-C library (DNA; Day 14, step 1) to the PCR machine, in the position marked in black below, and start the PCR program.

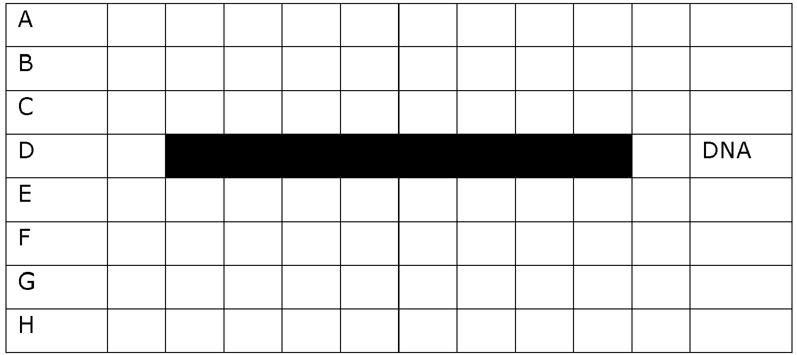

After just over 5 minutes (once the temperature has reached 65°C) transfer the PCR strip with the hybridization buffer (from Day 14, step 2) to the PCR machine, in the position marked in grey below. Incubate for 5 minutes.

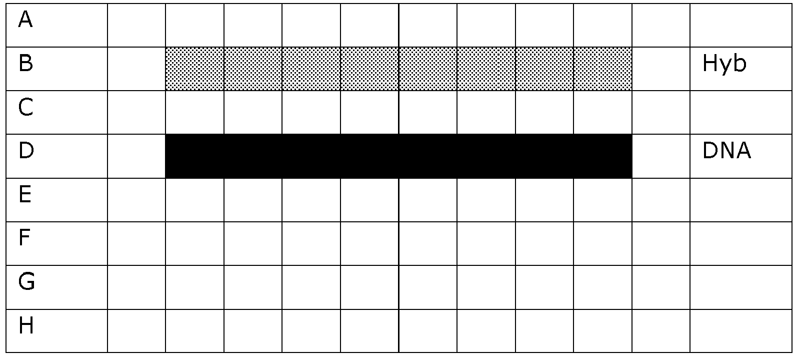

After 5 minutes (10 minutes since the start of the PCR program), transfer the PCR strip with the biotinylated RNA bait (see Day 14, step 3) to the PCR machine, in the position marked with cross-hatching below. Incubate for 2 minutes.

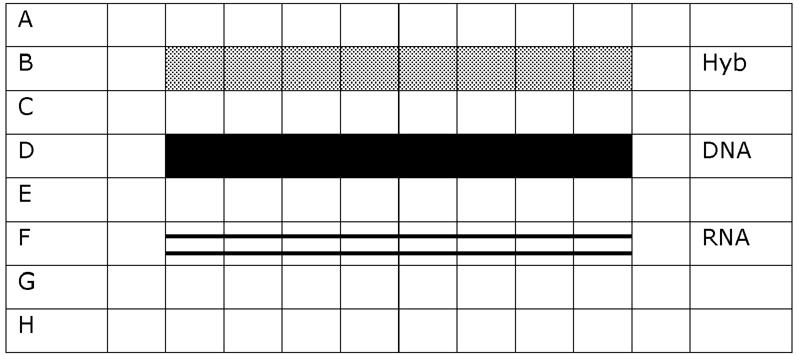

After 2 minutes, take off the lids from the PCR strip containing the hybridization buffer and the PCR strip containing the biotinylated RNA bait. Pipette 13µl of hybridization buffer into the 7µl of RNA bait (grey into cross-hatched). Discard the PCR strip containing the hybridization buffer. Proceed immediately to the next step.

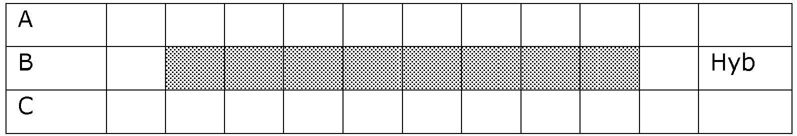

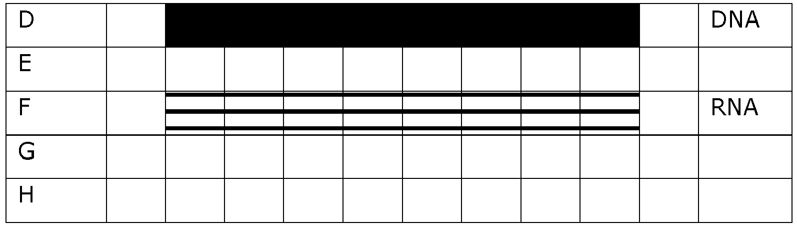

Take off the lid from the PCR strip containing the pond Hi-C library (DNA). Pipette 9µl of the Hi-C library (may be slightly less due to evaporation) into the 20µl of RNA bait with hybridization buffer (black into cross-hatched). Discard the empty PCR strip that contained the Hi-C library.

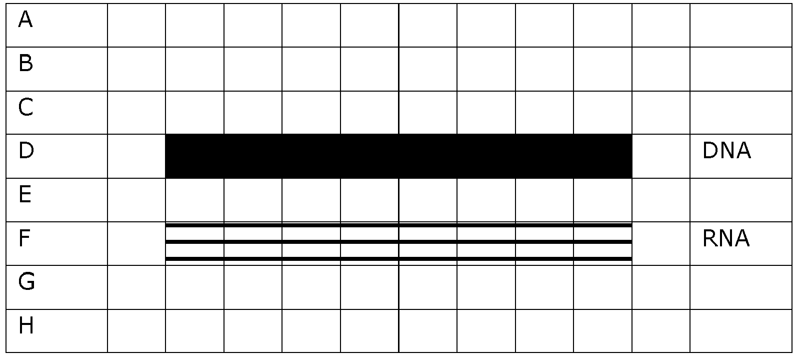

Close the remaining PCR strip (now containing Hi-C library/hybridization buffer/RNA bait) with a PCR strip tube lid (Agilent optical cap 8x strip 401425) immediately and incubate for 24 hours at 65°C.

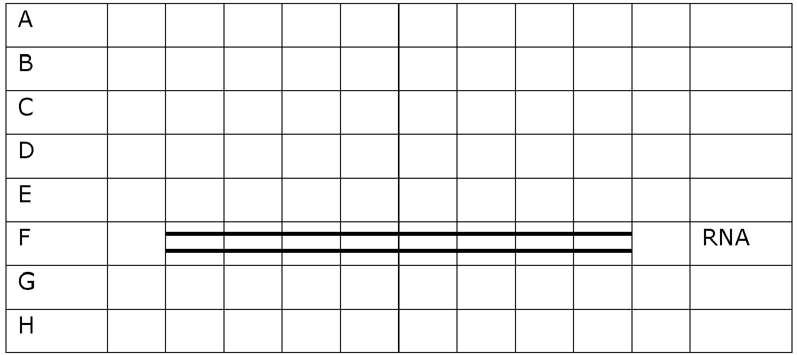

### Day 15: Streptavidin-biotin pulldown and washes

1. Prepare buffers:
   Binding buffer (BB, Agilent Technologies) at room temperature
   Wash buffer I (WB I, Agilent Technologies) at room temperature
   Wash buffer II (WB II Agilent Technologies) at 65°C
   NEB2 1x (NEB B7002S) at room temperature.
2. Wash magnetic beads: Mix Dynabeads MyOne Streptavidin T1 (Life Technologies 65601) thoroughly before adding 60µl per Capture Hi-C sample into a 1.5 ml lowbind Eppendorf tube. Wash the beads as follows (same procedures for all subsequent wash steps):
   a) Add 200µl BB
   b) Mix on vortex (at low to medium setting) for 5 seconds.
   c) Place tube on Dynal magnetic separator (Life Technologies)
   d) Reclaim beads, discard supernatant
   Repeat steps a) to d) for a total of 3 washes.
3. Biotin-Streptavidin pulldown: With the streptavidin beads in 200µl BB in a fresh low bind Eppendorf tube, open the lid of the PCR machine (while the PCR machine is running) and pipette the entire hybridization reaction into the tube containing the streptavidin beads.
   Incubate on a rotating wheel for 30 minutes at room temperature.
4. Washes: After 30 minutes, place the sample on the magnetic separator, discard supernatant, resuspend beads in 500µl WB I, and transfer to a fresh tube. Incubate at room temperature for 15 minutes. Vortex every 2 to 3 minutes for 5 seconds each.
   Separate the beads and buffer on a magnetic separator and remove the supernatant. Resuspend in 500µl WB II (prewarmed to 65°C) and transfer to a fresh tube.
   Incubate at 65°C for 10 minutes, and vortex (at low to medium setting) for 5 seconds every 2 to 3 minutes. Repeat for a total of 3 washes in WB II, all at 65°C.
5. After removing the supernatant, resuspend in 200µl 1xNEB2 and transfer immediately to a new tube. Put straight back onto the magnetic separator and remove supernatant.
6. Resuspend in 30µl 1xNEB2 and transfer to a fresh tube. The RNA/DNA mixture hybrid 'catch' on beads is now ready for PCR amplification.

### Day 16: Determine PCRs conditions for Capture Hi-C library amplification

1. To determine the optimal number of PCR cycles for Hi-C library, set up test PCRs with 6, 7, and 9 amplification cycles. Set up 3 reactions, each with:

| | |
|---|---|
| 2.5µl | Capture Hi-C library DNA on beads |
| 5µl | Buffer 5x (Phusion NEB F531) |
| 0.7µl | dATP 10mM |
| 0.7µl | dCTP 10mM |
| 0.7µl | dGTP 10mM |
| 0.7µl | dTTP 10mM |
| 0.075µl | PE PCR primer 1.0 (100µM) |
| 0.075µl | PE PCR primer 2.0 (100µM) |
| 0.3µl | Phusion polymerase (NEB F531) |
| 14.25µl | H₂O |

PE PCR Primer 1.0:
   AATGATACGGCGACCACCGAGATCTACACTCTTTCCCTACACGACGCTCTTCCGATCT (SEQ ID NO: 3)
PE PCR Primer 2.0:

2. Run 3 different PCRs with following conditions for n cycles:

| | | |
|---|---|---|
| 1 cycle: | 98°C | 30 seconds |
| | 65°C | 30 seconds |
| | 72°C | 30 seconds |
| n - 2 cycles: | 98°C | 10 seconds |
| | 65°C | 30 seconds |
| | 72°C | 30 seconds |
| 1 cycle: | 98°C | 10 seconds |
| | 65°C | 30 seconds |
| | 72°C | 7 minutes |

3. Check the amount of amplified DNA by running the entire reaction (25µl) on a 1.5% agarose gel. Typically, a smear in the range of 300 to 600 bp should be just about visible at 6 to 7 cycles of amplification.

### Day 17: Final PCR amplification of Capture Hi-C library

1. Set up x PCR reactions (remaining volume of Capture HiC library divided by factor 2.5) with 25µl each, as described on Day 16, step 1, with one PCR condition (*i.e.* chose number of PCR cycles). Typically, 4 PCR cycles should be sufficient to obtain Capture Hi-C library quantities suitable for Illumina sequencing.
2. Pool all individual PCR reactions from Day 17, step 1. Place on a magnetic separator, and transfer the supernatant into a fresh 1.5ml lowbind Eppendorf tube. Resuspend the streptavidin beads in the original amount of 1xNEBuffer 2 (see Day 15, step 6) and keep as a backup. Determine the volume of the Capture Hi-C library, and purify by adding 1.8x volume of SPRI beads (Beckman Coulter Ampure XP beads A63881), following the manufacturer's instructions. Resuspend in 100µl of TLE.
3. Repeat SPRI bead purification (as described on Day 17, step 2), by adding 180µl of SPRI beads to 100µl of Hi-C library from Day 17, step 2. Resuspend in a final volume of 20µl TLE.
4. Before sequencing, check the quality and quantity of the Capture Hi-C library by Bioanalyzer (Agilent) and quantitative PCR.

### EXAMPLE 2: Sequence Capture of Regions Interacting with Bait Loci (SCRiBL) Hi-C protocol

The present study describes the following three variants of SCRiBL Hi-C, two of which (b and c below) allow for barcoding and multiplexing of samples:
a) Conventional SCRiBL Hi-C (no barcoding)
b) Pre-capture barcoding SCRiBL Hi-C
c) Post-capture barcoding SCRiBL Hi-C

Follow Days 1 to 10 from the protocol in Example 1, then continue with the following steps:

### a) Conventional SCRiBL Hi-C:

Follow Days 11 to 13 from the protocol in Example 1, then continue with Step 14 below.

### b) Pre-capture barcoding SCRiBL Hi-C:

### Day 11: Biotin-streptavidin pulldown and adapter ligation

1. Use the Quant-iT PicoGreen (Life Technologies P7589) assay to determine the DNA yields. Prepare 1:20 and 1:50 dilutions for the sample(s). The expected yield from 40µg starting material is about 10µg.
   To avoid overloading the beads (see Day 11, step 4), it is recommended to not use more than a maximum of 2.5µg of DNA per 150µl of streptavidin beads.
2. Generate TruSeq adapters by annealing TruSeq universal adapter primer and TruSeq indexed adapter primer: (it should be noted that 'NNNNNN' is the barcode)
   5'-AATGATACGGCGACCACCGAGATCTACACTCTTTCCCTACACGACGCTCTTCCGATCT-3' (SEQ ID NO: 3)
   Mix 15µl of each TruSeq adapter primer with 70µl H₂O (total volume 100µl). In a PCR machine, run the following program: 95°C for 5 minutes, then decrease in temperature by 1°C per minute, until 4°C is reached.
   Make TruSeq adapter aliquots (15µM) of 10 to 20µl, store at -20°C, and thaw aliquot just before use.
3. Prepare buffers:
   a) TB (Tween buffer): 5mM Tris, 0.5mM EDTA, 1M NaCl, 0.05% Tween
      Prepare 1600µl per sample
   b) NTB (No Tween buffer): 5mM Tris, 0.5mM EDTA, 1M NaCl
      Prepare 600µl per sample
   c) NTB 2x: 10mM Tris, 1mM EDTA, 2M NaCl
      Prepare 300µl per sample
   d) Ligation buffer (10x ligation buffer NEB B0202S): 1x
      Prepare 150µl per sample
   e) NEBuffer 2: 1x
      Prepare 300µl per sample
4. Transfer 150µl of Dynabeads MyOne Streptavidin C1 beads (Life Technologies 650.01) suspension into a lowbind 1.5ml Eppendorf tube.
   Set up one reaction per 2µg to 2.5µg of DNA as determined on Day 11, step 1.
5. Wash beads twice with 400µl TB. Wash steps are always:
   a) Place sample on magnetic separator and reclaim beads
   b) Discard supernatant
   c) Add new buffer, mix thoroughly, and transfer sample to a new tube
   d) Rotate sample for 3 minutes at room temperature
   e) see a)
6. Resupend beads in 300µl of NTB 2x. Make up volume of Hi-C DNA (see Day 11, step 1) to 300µl with TLE.
   In case the amount of Hi-C library DNA exceeds 2.5µg (see Day 11, steps 1 and 4), prepare the adequate number of Hi-C samples, each containing a maximum of 2.5µg of DNA in a total volume of 300µl TLE.
   Combine the beads with the Hi-C DNA (total volume 600µl). Rotate slowly for 30 minutes at room temperature.
7. Place sample on magnetic separator, reclaim beads with bound Hi-C DNA, discard supernatant and wash once with 400µl NTB, followed by another wash with 200µl of 1x ligation buffer.
8. Resuspend in 50µl 1x ligation buffer and transfer to a fresh tube. Add 4µl of annealed 15µM TruSeq adapter (see Day 11, step 2), and 4µl of T4 DNA ligase (NEB M0202S). Rotate slowly at room temperature for 2 hours.
9. Place sample on magnetic separator, reclaim Hi-C bound beads, and wash twice with 400µl TB each.
10. Wash with 200µl 1xNTB, then wash with 200µl 1xNEBuffer 2.
11. Wash with 60µl 1xNEBuffer 2, then resuspend beads in 40µl 1xNEBuffer 2 and transfer into a fresh tube. If more than one streptavidin-biotin pulldown per Hi-C library was performed (*i.e.* if the starting amount of Hi-C library exceeded 2.5µg DNA, see Day 11, steps 1 and 4), pool all reactions. Store at 4°C.

### Day 12: Test PCRs to determine conditions for Hi-C library amplification

1. To determine the optimal number of PCR cycles for Hi-C library, set up test PCRs with 6, 9, 12, and 15 amplification cycles. Set up 4 reactions, each with:

| | |
|---|---|
| 2.5µl | Hi-C library DNA on beads |
| 5µl | Buffer 5x (Phusion NEB F531) |
| 0.7µl | dATP 10mM |
| 0.7µl | dCTP 10mM |
| 0.7µl | dGTP 10mM |
| 0.7µl | dTTP 10mM |
| 0.075µl | TruSeq PCR primer 1.0 (100µM) |
| 0.075µl | TruSeq PCR primer 2.0 (100µM) |
| 0.3µl | Phusion polymerase (NEB F531) |
| 14.25µl | H₂O |

TruSeq PCR Primer 1.0:
   AATGATACGGCGACCACCGAGATCTACACTCTTTCCCTACACGA
   (SEQ ID NO: 6)
TruSeq PCR Primer 2.0:
   CAAGCAGAAGACGGCATACGAGAT (SEQ ID NO: 7)

2. Run 4 different PCRs with following conditions for n cycles:

| | | |
|---|---|---|
| 1 cycle: | 98°C | 30 seconds |
| | 62°C | 30 seconds |
| | 72°C | 30 seconds |
| n - 2 cycles: | 98°C | 10 seconds |
| | 62°C | 30 seconds |
| | 72°C | 30 seconds |
| 1 cycle: | 98°C | 10 seconds |
| | 62°C | 30 seconds |
| | 72°C | 7 minutes |

3. Check the amount of amplified DNA by running the entire reaction (25µl) on a 1.5% agarose gel. Usually, a smear in the range of 300 to 600 bp should be just about visible at 9 (in some cases 6) cycles of amplification and increase in intensity with increasing number of PCR cycles.

### Day 13: Final PCR amplification of Hi-C library

1. Set up x PCR reactions (remaining volume of HiC library divided by factor 2.5) with 25µl each, as described on Day 12, step 1, with one PCR condition (*i.e.* number of cycles). The number of the PCR cycles will depend on the available amount of Hi-C library, and the test PCRs (Day 12), but typically 6 to 7 cycles should be sufficient to obtain approximately 500 ng of Hi-C library required for capture Hi-C (see below).
2. Pool all individual PCR reactions from Day 13, step 1. Place on a magnetic separator, and transfer the supernatant into a fresh 1.5ml lowbind Eppendorf tube. Resuspend the streptavidin beads in the original amount of 1xNEBuffer 2 (see Day 11, step 11) and keep as a backup. Determine the volume of the supernatant containing the amplified Hi-C library, and purify by adding 1.8x volume of SPRI beads (Beckman Coulter Ampure XP beads A63881), following the manufacturer's instructions. Resuspend in 100µl of TLE.
3. Repeat SPRI bead purification (as described on Day 13, step 2), by adding 180µl of SPRI beads to 100µl of Hi-C library from Day 13, step 2. Resuspend in a final volume of 20µl TLE.
4. Check the quality and quantity of the Hi-C library by Bioanalyzer (Agilent). Keep 50ng to 100ng of the Hi-C library back for next generation sequencing.

### Proceed to Day 14

### c) Post-capture barcoding SCRiBL Hi-C:

### Day 11: Biotin-streptavidin pulldown and adapter ligation

1. Use the Quant-iT PicoGreen (Life Technologies P7589) assay to determine the DNA yields. Prepare 1:20 and 1:50 dilutions for the sample(s). The expected yield from 40µg starting material is about 10µg.
   To avoid overloading the beads (see Day 11, step 4), it is recommended to not use more than a maximum of 2.5µg of DNA per 150µl of streptavidin beads.
2. Generate SCRiBL adapters by annealing SCRiBL adapter primers 1 and 2:
   5'-P-GATCGGAAGAGCACACGTCTGAACTCCAGTCAC-3' (SEQ ID NO: 8)
   5'-ACACTCTTTCCCTACACGACGCTCTTCCGATCT-3' (SEQ ID NO: 2)

   Mix 15µl of each SCRiBL adapter primer with 70µl H₂O (total volume 100µl). In a PCR machine, run the following program: 95°C for 5 minutes, then decrease in temperature by 1°C per minute, until 4°C is reached.
   Make SCRiBL adapter aliquots (15µM) of 10 to 20µl, store at -20°C, and thaw aliquot just before use.
3. Prepare buffers:
   a) TB (Tween buffer): 5mM Tris, 0.5mM EDTA, 1M NaCl, 0.05% Tween
      Prepare 1600µl per sample
   b) NTB (No Tween buffer): 5mM Tris, 0.5mM EDTA, 1M NaCl
      Prepare 600µl per sample
   c) NTB 2x: 10mM Tris, 1mM EDTA, 2M NaCl
      Prepare 300µl per sample
   d) Ligation buffer (10x ligation buffer NEB B0202S): 1x
      Prepare 150µl per sample
   e) NEBuffer 2: 1x
      Prepare 300µl per sample
4. Transfer 150µl of Dynabeads MyOne Streptavidin C1 beads (Life Technologies 650.01) suspension into a lowbind 1.5ml Eppendorf tube.
   Set up one reaction per 2µg to 2.5µg of DNA as determined on Day 11, step 1.
5. Wash beads twice with 400µl TB. Wash steps are always:
   a) Place sample on magnetic separator and reclaim beads
   b) Discard supernatant
   c) Add new buffer, mix thoroughly, and transfer sample to a new tube
   d) Rotate sample for 3 minutes at room temperature
   e) see a)
6. Resupend beads in 300µl of NTB 2x. Make up volume of Hi-C DNA (see Day 11, step 1) to 300µl with TLE.
   In case the amount of Hi-C library DNA exceeds 2.5µg (see Day 11, steps 1 and 4), prepare the adequate number of Hi-C samples, each containing a maximum of 2.5µg of DNA in a total volume of 300µl TLE.
   Combine the beads with the Hi-C DNA (total volume 600µl). Rotate slowly for 30 minutes at room temperature.
7. Place sample on magnetic separator, reclaim beads with bound Hi-C DNA, discard supernatant and wash once with 400µl NTB, followed by another wash with 200µl of 1x ligation buffer.
8. Resuspend in 50µl 1x ligation buffer and transfer to a fresh tube. Add 4µl of annealed 15µM SCRiBL adapter (see Day 11, step 2), and 4µl of T4 DNA ligase (NEB M0202S). Rotate slowly at room temperature for 2 hours.
9. Place sample on magnetic separator, reclaim Hi-C bound beads, and wash twice with 400µl TB each.
10. Wash with 200µl 1xNTB, then wash with 200µl 1xNEBuffer 2.
11. Wash with 60µl 1xNEBuffer 2, then resuspend beads in 40µl 1xNEBuffer 2 and transfer into a fresh tube. If more than one streptavidin-biotin pulldown per Hi-C library was performed (*i.e.* if the starting amount of Hi-C library exceeded 2.5µg DNA, see Day 11, steps 1 and 4), pool all reactions. Store at 4°C.

### Day 12: Test PCRs to determine conditions for Hi-C library amplification

1. To determine the optimal number of PCR cycles for Hi-C library, set up test PCRs with 6, 9, 12, and 15 amplification cycles. Set up 4 reactions, each with:

| | |
|---|---|
| 2.5µl | Hi-C library DNA on beads |
| 5µl | Buffer 5x (Phusion NEB F531) |
| 0.7µl | dATP 10mM |
| 0.7µl | dCTP 10mM |
| 0.7µl | dGTP 10mM |
| 0.7µl | dTTP 10mM |
| 0.075µl | SCRiBL pre-capture PCR primer 1.0 (100µM) |
| 0.075µl | SCRiBL pre-capture PCR primer 2.0 (100µM) |
| 0.3µl | Phusion polymerase (NEB F531) |
| 14.25µl | H₂O |

SCRiBL pre-capture PCR primer 1.0:
   ACACTCTTTCCCTACACGACGCTCTTCCGATCT (SEQ ID NO: 2)
SCRiBL pre-capture PCR primer 2.0:
   GTGACTGGAGTTCAGACGTGTGCTCTTCCGATC (SEQ ID NO: 9)

2. Run 4 different PCRs with following conditions for n cycles:

| | | |
|---|---|---|
| 1 cycle: | 98°C | 30 seconds |
| | 65°C | 30 seconds |
| | 72°C | 30 seconds |
| n - 2 cycles: | 98°C | 10 seconds |
| | 65°C | 30 seconds |
| | 72°C | 30 seconds |
| 1 cycle: | 98°C | 10 seconds |
| | 65°C | 30 seconds |
| | 72°C | 7 minutes |

3. Check the amount of amplified DNA by running the entire reaction (25µl) on a 1.5% agarose gel. Usually, a smear in the range of 300 to 600 bp should be just about visible at 9 (in some cases 6) cycles of amplification and increase in intensity with increasing number of PCR cycles.

### Day 13: Final PCR amplification of Hi-C library

1. Set up x PCR reactions (remaining volume of HiC library divided by factor 2.5) with 25µl each, as described on Day 12, step 1, with one PCR condition (*i.e.* number of cycles). The number of the PCR cycles will depend on the available amount of Hi-C library, and the test PCRs (Day 12), but typically 6 to 7 cycles should be sufficient to obtain approximately 500 ng of Hi-C library required for capture Hi-C (see below).
2. Pool all individual PCR reactions from Day 13, step 1. Place on a magnetic separator, and transfer the supernatant into a fresh 1.5ml lowbind Eppendorf tube. Resuspend the streptavidin beads in the original amount of 1xNEBuffer 2 (see Day 11, step 11) and keep as a backup. Determine the volume of the supernatant containing the amplified Hi-C library, and purify by adding 1.8x volume of SPRI beads (Beckman Coulter Ampure XP beads A63881), following the manufacturer's instructions. Resuspend in 100µl of TLE.
3. Repeat SPRI bead purification (as described on Day 13, step 2), by adding 180µl of SPRI beads to 100µl of Hi-C library from Day 13, step 2. Resuspend in a final volume of 20µl TLE.
4. Check the quality and quantity of the Hi-C library by Bioanalyzer (Agilent). Keep 50ng to 100ng of the Hi-C library back for next generation sequencing.

### Proceed to Day 14

***Generation of biotinylated RNA for target enrichment*** (for Conventional SCRiBL Hi-C, Pre-capture barcoding SCRiBL Hi-C, and Post-capture barcoding SCRiBL Hi-C)

### Day 14: Digestion of bacterial artificial chromosomes (BACs)

1. Chose and order bacterial artificial chromosomes (BACs) covering genomic regions of interest. Prepare BAC DNA using the Macherey Nagel NucleoBond BAC 100 kit (REF 740579), following the manufacturer's instructions.
2. Digest equimolar amounts of BAC DNA (20 to 25µg in total) with 400U HindIII in NEB buffer 2 (1x final) at 37°C overnight, in a total volume of 200 to 250µl.
   Note: the restriction enzyme used has to be the same as in Days 1 to 13 of Example 1 Hi-C library generation

### Day 15: Ligation of T7 promoter adapters to digested BAC DNA

1. Perform phenol chloroform extraction, followed by chloroform extraction. Precipitate with 0.1 volumes 3M Sodiumacetate and 2.5 volumes 100% ethanol for a minimum of two hours at -20°C (or overnight at -20°C).
2. Spin 14,000rpm for 20 minutes at 4°C. Wash with 800µl 70% ethanol, respin, remove ethanol and allow pellets to air dry.
3. Resuspend in 25µl 10mM Tris, pH7.5. Determine concentration by Nanodrop. The yield should be between 15 and 20µg DNA.
4. To prepare the T7 promoter adapter, mix 20µl each of both 'Luo T7 HindIII' and 'Luo T7 HindIII rc' primers (100µM) with 60µl oligo annealing buffer (10mM Tris, pH8.0; 50mM NaCl; 1mM EDTA), so that the final adapter concentration is 20µM.
   Use HPLC purified primers. Primer sequences can be found at the end of this part of the protocol.
5. Anneal adapters in a PCR machine: 95°C for 5 minutes, then decrease temperature at a rate of 1°C per minute, until 4°C is reached. The annealed adapters should be frozen in aliquots. Thaw on ice before the experiment and use immediately.
6. Add 15-fold molar excess of adapters (6.2µl assuming for 20µg of BAC DNA, approximately 4kb fragments) to DNA in 1X T4 DNA ligase buffer (NEB B0202S), 12µl (400U/µl) T4 DNA Ligase (NEB M0202S) in a final volume of 150µl. Incubate at 16°C overnight.

### Day 16: Sonication of digested T7 promoter-DNA

1. Inactivate T4 DNA ligase by heating at 65°C for 10 minutes, cool on ice.
2. The DNA (BAC HindIII fragments ligated to T7 promoter) will now be in 150µl. Add 120µl H₂O to make 270µl. Remove 10µl to run on gel (pre-sonication sample) and split the remaining volume into two samples of 130µl each (the volume required for the sonication by Covaris E220).
3. Pipette into metal-lidded covaris naked DNA tubes through septum, avoid generating bubbles that may interfere with sonication.
4. Use the following Covaris E220 conditions to generate fragments with a peak at 200 base pairs:
   10% duty factor
   175W peak incident power
   200 cycles per burst
   180 seconds treatment time
   7°C
5. After sonication, transfer the sample from the covaris tubes into a fresh Eppendorf tube.
6. Optional: Run pre- and post-sonication aliquots on a 2% agarose gel to assess fragmentation.

### Day 17: End repair and SPRI bead size selection

1. The recovered volume of sonicated DNA should be 130µl per sample. Add 17µl 2.5mM mixed dNTPs, 9.5µl 10X NEB Ligase buffer (NEB B0202S) making final concentration 1x, 6µl T4 DNA Polymerase (NEB M0203L), 6µl Polynucleotide Kinase (NEB M0201L), 1.5µl Klenow (NEB M0210L), to a final volume of 170µl per reaction. Incubate at room temperature for 30 minutes.
2. Separate each of the two 170µl reactions in two Eppendorf tubes containing 85µl each (to avoid overloading the Qiagen purification columns in the subsequent steps). Purify the DNA in all four tubes using the Qiagen PCR purification kit (Qiagen 28104), elute with 50µl each, and then combine two samples each after the final elution (total volume at the end should be 100µl for two samples in total).
3. Perform a double-sided SPRI bead (Agencourt AMPure XP beads A63881) size selection (0.7x, followed by 1.0x) to isolate BAC-T7 promoter DNA in the size range of 180 to 300 bp. The recovered volume of sonicated and repaired DNA should be 100µl now (tube A). Mix SPRI beads well by vortexing, and transfer 180µl of SPRI beads into a fresh Eppendorf tube (tube B). Add 70µl of these 180µl of SPRI beads to the 100µl of DNA in tube (A) (0.7X), mix thoroughly, incubate for 10 minutes at room temperature, and place on magnetic separator and recover the unbound supernatant containing the DNA in the desired size range into a fresh tube (tube C). Discard tube (A) containing the beads.
4. This step is to concentrate the beads. Place tube (B) (containing 110µl of SPRI beads) on the magnet, and remove all but 30µl of the supernatant (*i.e.* discard around 80µl of the supernatant). Take tube (B) off the magnet and resuspend the beads in the remaining 30µl volume.
5. Add 30µl of concentrated beads from tube (B) into tube (C) (1.0x SPRI bead, *i.e.* ratio of DNA in solution to SPRI beads in solution is now 1:1). Mix well, incubate 10 minutes at room temperature, place on magnet, and discard supernatant. Discard tube (B).
6. Resuspend bead-bound DNA in tube (C) in 25µl TLE (Tris low-EDTA: 10mM Tris, 0.1mM EDTA), incubate at room temperature for 5 minutes, place on magnet and transfer supernatant (containing your size-selected DNA) into a fresh tube (D). Discard tube (C) containing the beads.
7. Determine the concentration of the size-selected DNA.

### Day 18: In vitro transcription with biotin-UTP

1. Use the T7 MegaScript kit (Ambion; AM1333) with either Biotin RNA labeling mix (Roche; Cat. No. 11 685 597 910), or (cheaper option) with biotin-UTP (Roche; Cat. No. 11 388 908 910) and mix with standard rNTPs. Set up the following reaction:
   2µl 10X buffer
   5.5µl DNA template (typically between 100 and 500 ng)
   5µl biotin-UTP (Roche 11 388 908 910 stock is 10 mM)
   1µl unlabelled rUTP (100 mM stock)
   1.5µl rATP (100 mM stock)
   1.5µl rCTP (100 mM stock)
   1.5µl rGTP (100 mM stock)
   2µl enzyme mix
   Note: This results in a final nucleotide concentration of 7.5 mM for each nucleotide, with a ratio of labelled to unlabelled UTP of 1:2.
2. Incubate the reaction at 37°C overnight (12 to 16 hours).

### Day 19: Purification of biotinylated RNA

1. To remove the template DNA, treat with 1µl Turbo DNAse (Life Technologies AM2238) for 15 minutes at 37°C.
2. Purify the RNA using the Ambion MEGAclear kit (AM1908M) following the manufacturer's instructions. Elute in 50µl final volume. Make 5 to 10µl aliquots and freeze as quickly as possible. The expected yield will typically be between 20µg and 50µg RNA, as determined by Nanodrop. This amount is sufficient for 40 to 100 individual SCRiBL Hi-C reactions (using 500ng of biotinylated RNA bait per experiment).
3. Check the size and integrity of the obtained RNA by running 1 to 2µg of RNA on a 2% agarose gel (use formamide loading dye provided with Ambion MegaScript kit).

Adapter Primers (HindIII 5'-overhang underlined, modify accordingly when using other restriction enzymes):
Luo T7 HindIII:
   5'-TCTAGTCGACGGCCAGTGAATTGTAATACGACTCACTATAGGGCGA-3' (SEQ ID NO: 10)
LuoT7 HindIII rc:

### Solution hybrid capture of Hi-C library

### Day 20: Hybridization of Hi-C library with biotin-RNA target bait

1. Prepare Hi-C library (pond): transfer volume equivalent to 500ng of Hi-C library (Day 13, step 4 of Example 1) into a 1.5ml lowbind Eppendorf tube and concentrate using a SpeedVac.
   Keep 50ng to 100ng Hi-C library back for next generation sequencing.
   After evaporation of all liquid, resuspend the Hi-C DNA pellet in 3.5µl of H₂O and transfer into a fresh 1.5 ml lowbind tube (per single SCRiBL reaction). Add the following components:
   2.5µl cot1 DNA (mouse cot-1 DNA Invitrogen 18440-016, 1mg/ml; 2.5 µg total)
   1µl salmon sperm DNA (sheared, Ambion AM9680 10 mg/ml stock, make 1:4 dilution (2.5 mg/ml), so that final amount added is 2.5µg)

Add the following blocking oligos:

### a) Conventional SCRiBL Hi-C:

For Hi-C libraries generated using standard PE adapters and PE PCR 1.0 and PE PCR 2.0 for amplification, use the following two blocking oligos:
1.5µl PE blocking oligo 1 (200 µM stock; identical in sequence to PE PCR 1.0):
   AATGATACGGCGACCACCGAGATCTACACTCTTTCCCTACACGACGCTCTTCCGATCT (SEQ ID NO: 3)
1.5µl PE blocking oligo 2 (200 µM stock; identical in sequence to PE PCR 2.0):

### b) Pre-capture barcoding SCRiBL Hi-C:

For Hi-C libraries generated using Illumina TruSeq adapters, and PCR-amplified with TruSeq PCR primer 1.0 and TruSeq PCR primer 2.0, use the following two blocking oligos:
1.5µl TruSeq universal blocking oligo (200 µM stock; identical in sequence to TruSeq universal adapter):
   AATGATACGGCGACCACCGAGATCTACACTCTTTCCCTACACGACGCTCTTCCGATCT (SEQ ID NO: 3)
1.5µl TruSeq blocking oligo index x (200 µM stock; reverse complement of TruSeq adapter index x):
   Note: use the Truseq blocking oligo index corresponding to the TruSeq adapter index used to generate the Hi-C library. For example, if you have used TruSeq adapter index 6 for Hi-C library generation:
   In this case, use TruSeq blocking oligo index 6 (reverse complement of TruSeq adapter index 6) as blocking oligo:

### c) Post-capture barcoding SCRiBL Hi-C:

For Hi-C libraries generated using SCRiBL adapters, and PCR-amplified with SCRiBL pre-capture PCR primer 1.0 and SCRiBL pre-capture PCR primer 2.0, use the following four blocking oligos:
0.75µl SCRiBL blocking oligo 1 (200 µM stock):

| | |
|---|---|
| GTGACTGGAGTTCAGACGTGTGCTCTTCCGATCdd* | (SEQ ID NO: 15) |

0.75µl SCRiBL blocking oligo 2 (200 µM stock):

| | |
|---|---|
| AGATCGGAAGAGCGTCGTGTAGGGAAAGAGTGT | (SEQ ID NO: 16) |

0.75µl SCRiBL blocking oligo 3 (200 µM stock):

| | |
|---|---|
| AGATCGGAAGAGCACACGTCTGAACTCCAGTCAC | (SEQ ID NO: 17) |

0.75µl SCRiBL blocking oligo 4 (200 µM stock):

| | |
|---|---|
| ACACTCTTTCCCTACACGACGCTCTTCCGATCdd* | (SEQ ID NO: 18) |

| | |
|---|---|
| * SCRiBL blocking oligos 1 and 4 contain a 3' didesoxy modification | |

Resuspend thoroughly, transfer into PCR strip (Agilent 410022), close with a PCR strip tube lid (Agilent optical cap 8x strip 401425) and keep on ice.
2. Prepare 2.23x hybridization buffer (30µl per SCRiBL Hi-C sample):

| | |
|---|---|
| 11.15x SSPE | (stock 20x; Gibco 15591-043) |
| 11.15x Denhardt's | (stock 50x; Invitrogen 750018) |
| 11.15mM EDTA | (stock 500mM; Gibco 15575-038) |
| 0.223% SDS | (stock 10%; Promega V6551) |

Mix thoroughly, heat to 65°C for 5 minutes, transfer into PCR strip (Agilent 410022), close with a PCR strip tube lid (Agilent optical cap 8x strip 401425) and keep at room temperature.
3. Prepare biotin-RNA (bait): transfer 500ng of biotinylated RNA (Day 19, step 2) into a 1.5ml lowbind Eppendorf tube, and make up to a volume of 5.5µl with H₂O. Add 1.5µl of SUPERase-In (Ambion AM2694, 20 units/µl). Transfer into a PCR strip (Agilent 410022), close with a PCR strip tube lid (Agilent optical cap 8x strip 401425) and keep on ice (volume should now be 7µl).
4. Hybridization reaction: Set the PCR machine (PTC-200, MJ Research) to the following program (PCR machine lid must be heated throughout):
1. 95°C for 5 minutes
2. 65°C forever

Follow the same instructions for transferring the PCR strip as in Day 14, step 4 of Example 1.

### Day 21: Streptavidin-biotin pulldown and washes

1. Prepare buffers:
   a) Binding buffer (BB):
      1M NaCl
      10mM Tris-HCI pH 7.5
      1mM EDTA
      Prepare 800µl per SCRiBL sample and keep at room temperature
   b) Wash buffer I (WB I):
      1xSSC (SSC 20x stock: Invitrogen 15557-044)
      0.1% SDS
      Prepare 500µl per SCRiBL sample and keep at room temperature
   c) Wash buffer II (WB II):
      0.1xSSC (SSC 20x stock: Invitrogen 15557-044)
      0.1% SDS
      Prepare 1500µl per SCRiBL sample, heat to 65°C and keep at this temperature
   d) NEB2 1x (10x stock; NEB B7002S)
      Prepare 230µl per SCRiBL sample and keep at room temperature
2. Wash magnetic beads: Mix Dynabeads MyOne Streptavidin T1 (Life Technologies 65601) thoroughly before adding 60µl per SCRiBL Hi-C sample into a 1.5 ml lowbind Eppendorf tube. Wash the beads as follows (same procedures for all subsequent wash steps):
   a) Add 200µl BB
   b) Mix on vortex (at low to medium setting) for 5 seconds.
   c) Place tube on Dynal magnetic separator (Life Technologies)
   d) Reclaim beads, discard supernatant
   Repeat steps a) to d) for a total of 3 washes.
3. Biotin-Streptavidin pulldown: With the streptavidin beads in 200µl BB in a fresh low bind Eppendorf tube, open the lid of the PCR machine (while the PCR machine is still running), pipette the entire hybridization reaction into the tube containing the streptavidin beads and mix well.
   Check the volume of the hybridization reaction at this point. A volume below 20µl means that excessive evaporation is likely to have resulted in suboptimal hybridization conditions.
   Incubate on a rotating wheel for 30 minutes at room temperature.
4. Washes: After 30 minutes, place the sample on the magnetic separator, discard supernatant, resuspend beads in 500µl WB I, and transfer to a fresh tube. Incubate at room temperature for 15 minutes. Vortex every 2 to 3 minutes for 5 seconds each.
   Separate the beads and buffer on a magnetic separator and remove the supernatant. Resuspend in 500µl WB II (pre-warmed to 65°C) and transfer to a fresh tube. Incubate at 65°C for 10 minutes, and vortex (at low to medium setting) for 5 seconds every 2 to 3 minutes. Repeat for a total of 3 washes in WB II, all at 65°C.
5. After removing the supernatant, resuspend in 200µl 1xNEB2 and transfer immediately to a new tube. Put straight back onto the magnetic separator and remove supernatant.
6. Resuspend in 30µl 1xNEB2 and transfer to a fresh tube. The RNA/DNA mixture hybrid 'catch' on beads is now ready for PCR amplification.

### Day 22: Determine PCRs conditions for SCRiBL Hi-C library amplification

### a) Conventional SCRiBL Hi-C:

1. To determine the optimal number of PCR cycles for SCRiBL Hi-C library amplification, set up test PCRs with 7, 9, and 12 amplification cycles. Set up 3 reactions, each with:

| | |
|---|---|
| 2.5µl | SCRiBL Hi-C library DNA on beads |
| 5µl | Buffer 5x (Phusion NEB F531) |
| 0.7µl | dATP 10mM |
| 0.7µl | dCTP 10mM |
| 0.7µl | dGTP 10mM |
| 0.7µl | dTTP 10mM |
| 0.075µl | PE PCR primer 1.0 (100µM) |
| 0.075µl | PE PCR primer 2.0 (100µM) |
| 0.3µl | Phusion polymerase (NEB F531) |
| 14.25µl | H₂O |

PE PCR Primer 1.0:
   AATGATACGGCGACCACCGAGATCTACACTCTTTCCCTACACGACGCTCTTCCGATCT (SEQ ID NO: 3)
PE PCR Primer 2.0:

2. Run 3 different PCRs with following conditions for n cycles:

| | | |
|---|---|---|
| 1 cycle: | 98°C | 30 seconds |
| | 65°C | 30 seconds |
| | 72°C | 30 seconds |
| n - 2 cycles: | 98°C | 10 seconds |
| | 65°C | 30 seconds |
| | 72°C | 30 seconds |
| 1 cycle: | 98°C | 10 seconds |
| | 65°C | 30 seconds |
| | 72°C | 7 minutes |

3. Check the amount of amplified DNA by running the entire reaction (25µl) on a 1.5% agarose gel. Typically, a smear in the range of 300 to 600 bp should be just about visible at 9 cycles of amplification.

### Day 22: Determine PCRs conditions for SCRiBL Hi-C library amplification

### b) Pre-capture barcoding SCRiBL Hi-C

1. To determine the optimal number of PCR cycles for SCRiBL Hi-C library amplification, set up test PCRs with 7, 9, and 12 amplification cycles. Set up 3 reactions, each with:

| | |
|---|---|
| 2.5µl | SCRiBL Hi-C library DNA on beads |
| 5µl | Buffer 5x (Phusion NEB F531) |
| 0.7µl | dATP 10mM |
| 0.7µl | dCTP 10mM |
| 0.7µl | dGTP 10mM |
| 0.7µl | dTTP 10mM |
| 0.075µl | TruSeq PCR primer 1.0 (100µM) |
| 0.075µl | TruSeq PCR primer 2.0 (100µM) |
| 0.3µl | Phusion polymerase (NEB F531) |
| 14.25µl | H₂O |

2. Run 3 different PCRs with following conditions for n cycles:

| | | |
|---|---|---|
| 1 cycle: | 98°C | 30 seconds |
| | 62°C | 30 seconds |
| | 72°C | 30 seconds |
| n - 2 cycles: | 98°C | 10 seconds |
| | 62°C | 30 seconds |
| | 72°C | 30 seconds |
| 1 cycle: | 98°C | 10 seconds |
| | 62°C | 30 seconds |
| | 72°C | 7 minutes |

3. Check the amount of amplified DNA by running the entire reaction (25µl) on a 1.5% agarose gel. Typically, a smear in the range of 300 to 600 bp should be just about visible at 9 cycles of amplification.

### Day 22: Determine PCRs conditions for SCRiBL Hi-C library amplification

### c) Post-capture barcoding SCRiBL Hi-C

1. To determine the optimal number of PCR cycles for SCRiBL Hi-C library amplification, set up test PCRs with 7, 9, and 12 amplification cycles. Set up 3 reactions, each with:

| | |
|---|---|
| 2.5µl | SCRiBL Hi-C library DNA on beads |
| 5µl | Buffer 5x (Phusion NEB F531) |
| 0.7µl | dATP 10mM |
| 0.7µl | dCTP 10mM |
| 0.7µl | dGTP 10mM |
| 0.7µl | dTTP 10mM |
| 0.075µl | SCRiBL post-capture PCR primer 1.0 (100µM) |
| 0.075µl | SCRiBL post-capturePCR primer 2.0 (100µM) |
| 0.3µl | Phusion polymerase (NEB F531) |
| 14.25µl | H₂O |

SCRiBL post-capture PCR primer 1.0
   AATGATACGGCGACCACCGAGATCTACACTCTTTCCCTACACGACGCTCTTCCGATCT (SEQ ID NO: 3)
SCRiBL post-capture PCR primer 2.0: (please note that 'NNNNNN' is the barcode)

2. Run 3 different PCRs with following conditions for n cycles:

| | | |
|---|---|---|
| 1 cycle: | 98°C | 30 seconds |
| | 65°C | 30 seconds |
| | 72°C | 30 seconds |
| n - 2 cycles: | 98°C | 10 seconds |
| | 65°C | 30 seconds |
| | 72°C | 30 seconds |
| 1 cycle: | 98°C | 10 seconds |
| | 65°C | 30 seconds |
| | 72°C | 7 minutes |

3. Check the amount of amplified DNA by running the entire reaction (25µl) on a 1.5% agarose gel. Typically, a smear in the range of 300 to 600 bp should be just about visible at 9 cycles of amplification.

### Day 23: Final PCR amplification of SCRiBL Hi-C library

1. Set up x PCR reactions (x equals the remaining volume of SCRiBL HiC library divided by factor 2.5) with 25µl each, as described above (see Day 22, step 1), with one PCR condition (*i.e.* chose number of PCR cycles). Typically, 6 PCR cycles should be sufficient to obtain SCRiBL Hi-C library quantities suitable for Illumina sequencing.
2. Pool all individual PCR reactions from Day 23, step 1. Place on a magnetic separator, and transfer the supernatant into a fresh 1.5ml lowbind Eppendorf tube. Resuspend the streptavidin beads in the original amount of 1xNEBuffer 2 (see Day 21, step 6) and keep as a backup. Determine the volume of the supernatant containing the SCRiBL Hi-C library, and purify by adding 1.8x volume of SPRI beads (Beckman Coulter Ampure XP beads A63881), following the manufacturer's instructions. Resuspend in 100µl of TLE.
3. Repeat SPRI bead purification (as described on Day 23, step 2), by adding 180µl of SPRI beads to 100µl of Hi-C library from Day 23, step 2. Resuspend in a final volume of 20µl TLE.
4. Before sequencing, check the quality and quantity of the SCRiBL Hi-C library by Bioanalyzer (Agilent) and quantitative PCR.

### EXAMPLE 3: Investigation of MYC gene Promoter Interactions

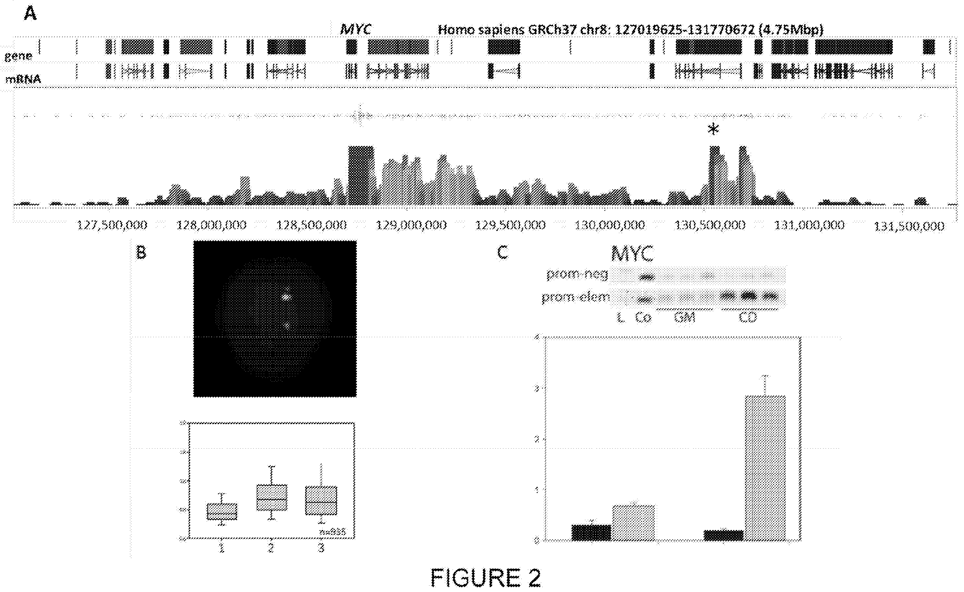

The method described herein allows the capture of the chromosomal interactions for thousands of promoters simultaneously, which can subsequently be analyzed separately. As an example, the genomic interactions surrounding the MYC gene are shown herein. The results may be explained with reference to **Figure 2** as shown herein.

**Figure 2(A)** displays the genomic region surrounding the MYC gene. **Figure 2(A)** shows the locations of regions that interact with the MYC promoter in CD34+ haematopoietic progenitor cells, as identified by the method of the invention. The asterisk shows the position of an interacting region that is located 1.8 Mb downstream of the gene that was corroborated as described below.

**Figure 2(B)** shows an example of a DNA FISH of a CD34+ cell nucleus. In these experiments, fluorescently labelled DNA probes that are specific to regions in the MYC locus (locations shown as bars in **Figure 2(A)****)** were hybridised to the genome in fixed CD34+ cell nuclei. The relative separation distances between these probes were measured for 935 loci. Quantitation of the distances is shown as a box and whiskers graph. Column 1 shows the separation distance between the MYC promoter and the distal interacting region. Its separation distance is significantly closer than the MYC promoter and an intervening region that did not show interactions in the method of the invention, or the intervening region and the distal interacting region, as shown by columns 2 and 3, respectively. P-value < 0.001.

**Figure 2(C)** shows validation of the interaction between the MYC promoter and the region at 1.8 Mb downstream of the gene using the known 3C method. Specific primers to the MYC promoter, the distal interacting element and the intervening region were used to amplify in three independently prepared 3C interaction libraries from CD34+ cells and GM12878 cells, in which the interaction was not detected in the method of the invention. The amplification products from a limited-cycle PCR were run on an agarose gel. L, DNA ladder marker; Co, control amplification product; GM, amplification from the three GM12878 3C libraries; CD, amplification from the three CD34+ 3C libraries. The graph in Figure 2(C) shows the quantitation of the gels, normalised against the control amplification product, of the MYC promoter interaction with the intervening region (black bars) and the interacting region (grey bars) for GM12878 cells (left) and CD34+ cells (right).

The data presented in Figure 2(B) and 2(C) thus serve as validation of the findings shown in Figure 2(A). Together, these results suggest that the region located 1.8 Mb downstream of the MYC gene interacts with the MYC promoter at significantly higher frequencies than a region that is much closer on the linear genome sequence, as was predicted by the method of the invention.

## Claims

1. A method for identifying nucleic acid segments which interact with a sub-group of target nucleic acid segments, said method comprising the steps of:
crosslinking a nucleic acid composition comprising the sub-group of target nucleic acid segments;
fragmenting the crosslinked nucleic acid composition;
marking the ends of the fragments with biotin;
ligating the fragmented nucleic acid segments to produce ligated fragments;
reversing the crosslinking;
shearing the fragments followed by end-repair;
pulldown of fragments with streptavidin;
adapter ligation followed by PCR;
promoter capture by addition of isolating nucleic acid molecules which bind to the sub-group of target nucleic acid segments, wherein said isolating nucleic acid molecules are labelled with a first half of a binding pair;
isolating ligated fragments which contain the sub-group of target nucleic acid segments bound to the isolating nucleic acid molecules by using the second half of the binding pair;
amplification using PCR; and
sequencing to identify the nucleic acid segments which interact with the sub-group of target nucleic acid segments.

2. The method of claim 1, wherein the sub-group of target nucleic acid molecules comprise a regulatory element selected from an enhancer, silencer or insulator.

3. The method of claim 1 or claim 2, wherein the isolating nucleic acid molecules are obtained from bacterial artificial chromosomes (BACs), fosmids or cosmids.

4. The method of any one of claims 1 to 3, wherein the isolating nucleic acid molecules are RNA.

5. The method of any one of claims 1 to 4, wherein the first half of the binding pair comprises biotin and the second half of the binding pair comprises streptavidin.

6. The method of any one of claims 1 to 5, wherein said nucleic acid composition is derived from a mammalian cell nucleus, such as a human cell nucleus.

7. The method of any one of claims 1 to 5, wherein said nucleic acid composition is derived from a non-human cell nucleus, such as a mouse cell nucleus or a plant cell nucleus.

8. A method of identifying one or more interacting nucleic acid segments that are indicative of a particular disease state comprising:
a) performing the method of any one of claims 1 to 7 on a nucleic acid composition obtained from an individual with a particular disease state;
b) quantifying a frequency of interaction between a nucleic acid segment and a target nucleic acid segment;
c) comparing the frequency of interaction in the nucleic acid composition from the individual with said disease state with the frequency of interaction in a normal control nuclear composition from a healthy subject, such that a difference in the frequency of interaction in the nucleic acid composition is indicative of a particular disease state.

9. The method of claim 8, wherein the disease state is selected from cancer, an autoimmune disease, a developmental disease or a genetic disorder.

10. Use of a kit for identifying nucleic acid segments which interact with a sub-group of target nucleic acid segments, which comprises buffers and reagents capable of performing the method of any one of claims 1 to 7.

## Patentansprüche

1. Verfahren zum Identifizieren von Nukleinsäuresegmenten, die mit einer Untergruppe von Zielnukleinsäuresegmenten interagieren, wobei das Verfahren folgende Schritte umfasst:
Vernetzen einer Nukleinsäurezusammensetzung, die die Untergruppe von Zielnukleinsäuresegmenten umfasst;
Fragmentieren der vernetzten Nukleinsäurezusammensetzung;
Markieren der Enden der Fragmente mit Biotin;
Ligieren der fragmentierten Nukleinsäuresegmente zur Herstellung von ligierten Fragmenten;
Umkehren der Vernetzung;
Scheren der Fragmente, gefolgt von einer Endreparatur;
Pulldown von Fragmenten mit Streptavidin;
Adapterligation, gefolgt von PCR;
Einfangen von Promotoren durch Zugabe von isolierenden Nukleinsäuremolekülen, die sich an die Untergruppe von Zielnukleinsäuresegmenten binden, wobei die isolierenden Nukleinsäuremoleküle mit einer ersten Hälfte eines Bindungspaares markiert sind;
Isolieren von ligierten Fragmenten, die die Untergruppen von Zielnukleinsäuresegmenten enthalten, welche unter Verwendung der zweiten Hälfte des Bindungspaares an die isolierenden Nukleinsäuremoleküle gebunden sind;
Amplifikation unter Verwendung von PCR; und
Sequenzieren, um die Nukleinsäuresegmente zu identifizieren, die mit der Untergruppe von Zielnukleinsäuresegmenten interagieren.

2. Verfahren nach Anspruch 1, wobei die Untergruppe von Zielnukleinsäuremolekülen ein regulatorisches Element umfasst, das aus einem Enhancer, Silencer oder Insulator ausgewählt ist.

3. Verfahren nach Anspruch 1 oder 2, wobei die isolierenden Nukleinsäuremoleküle aus künstlichen Bakterienchromosomen (BAC), Fosmiden oder Cosmiden erhalten werden.

4. Verfahren nach einem der Ansprüche 1 bis 3, wobei die isolierenden Nukleinsäuremoleküle RNA sind.

5. Verfahren nach einem der Ansprüche 1 bis 4, wobei die erste Hälfte des Bindungspaares Biotin umfasst und die zweite Hälfte des Bindungspaares Streptavidin umfasst.

6. Verfahren nach einem der Ansprüche 1 bis 5, wobei die Nukleinsäurezusammensetzung von einem Säugetier-Zellkern, wie etwa einem menschlichen Zellkern, abgeleitet ist.

7. Verfahren nach einem der Ansprüche 1 bis 5, wobei die Nukleinsäurezusammensetzung von einem nicht-menschlichen Zellkern, wie etwa einem Maus-Zellkern oder einem pflanzlichen Zellkern, abgeleitet ist.

8. Verfahren zum Identifizieren eines oder mehrerer interagierender Nukleinsäuresegmente, die einen bestimmten Krankheitszustand anzeigen, das Folgendes umfasst:
a) Durchführen des Verfahrens nach einem der Ansprüche 1 bis 7 an einer Nukleinsäurezusammensetzung, die von einem Individuum mit einem bestimmten Krankheitszustand erhalten wurde;
b) Quantifizieren einer Interaktionshäufigkeit zwischen einem Nukleinsäuresegment und einem Zielnukleinsäuresegment;
c) Vergleich der Interaktionshäufigkeit in der Nukleinsäurezusammensetzung vom Individuum mit dem Krankheitszustand mit der Interaktionshäufigkeit in einer normalen Kontroll-Nuklearzusammensetzung von einem gesunden Subjekt, so dass ein Unterschied in der Interaktionshäufigkeit in der Nukleinsäurezusammensetzung einen bestimmten Krankheitszustand anzeigt.

9. Verfahren nach Anspruch 8, wobei der Krankheitszustand aus Krebs, einer Autoimmunkrankheit, einer Entwicklungskrankheit oder einer genetischen Störung ausgewählt ist.

10. Verwendung eines Kits zum Identifizieren von Nukleinsäuresegmenten, die mit einer Untergruppe von Zielnukleinsäuresegmenten interagieren, das Puffer und Reagenzien umfasst, die das Verfahren nach einem der Ansprüche 1 bis 7 durchführen können.

## Revendications

1. Procédé pour identifier des segments d'acide nucléique qui interagissent avec un sous-groupe de segments d'acide nucléique cibles, ledit procédé comprenant les étapes consistant à :
réticuler une composition d'acide nucléique comprenant le sous-groupe de segments d'acide nucléique cibles ;
fragmenter la composition d'acide nucléique réticulée ;
marquer les extrémités des fragments avec de la biotine ;
ligaturer les segments d'acide nucléique fragmentés pour produire des fragments ligaturés ;
inverser la réticulation ;
cisailler les fragments avant une réparation des extrémités ;
isoler les fragments avec de la streptavidine ;
ligaturer l'adaptateur avant une PCR ;
capturer le promoteur par l'addition de molécules d'acide nucléique isolantes qui se lient au sous-groupe de segments d'acide nucléique cibles, dans lequel lesdites molécules d'acide nucléique isolantes sont marquées avec une première moitié d'une paire de liaison ;
isoler les fragments ligaturés qui contiennent le sous-groupe de segments d'acide nucléique cibles liés aux molécules d'acide nucléique isolantes en utilisant la seconde moitié de la paire de liaison ;
amplifier en utilisant une PCR ; et
séquencer pour identifier les fragments d'acide nucléique qui interagissent avec le sous-groupe de segments d'acide nucléique cibles.

2. Procédé selon la revendication 1, dans lequel le sous-groupe de molécules d'acide nucléique cibles comprend un élément régulateur sélectionné parmi une séquence stimulatrice, une séquence extinctrice ou une séquence isolante.

3. Procédé selon la revendication 1 ou la revendication 2, dans lequel les molécules d'acide nucléique isolantes sont obtenues parmi des chromosomes bactériens artificiels (CBA), des fosmides ou des cosmides.

4. Procédé selon l'une quelconque des revendications 1 à 3, dans lequel les molécules d'acide nucléique isolantes sont l'ARN.

5. Procédé selon l'une quelconque des revendications 1 à 4, dans lequel la première moitié de la paire de liaison comprend de la biotine et la seconde moitié de la paire de liaison comprend de la streptavidine.

6. Procédé selon l'une quelconque des revendications 1 à 5, dans lequel ladite composition d'acide nucléique est dérivée d'un noyau de cellule de mammifère, tel qu'un noyau de cellule humaine.

7. Procédé selon l'une quelconque des revendications 1 à 5, dans lequel ladite composition d'acide nucléique est dérivée d'un noyau de cellule non humaine, tel qu'un noyau de cellule de souris ou un noyau de cellule de végétal.

8. Procédé d'identification d'un ou plusieurs segments d'acide nucléique en interaction qui sont indicatifs d'un état pathologique particulier comprenant :
a) la réalisation du procédé selon l'une quelconque des revendications 1 à 7 sur une composition d'acide nucléique obtenue à partir d'un individu avec un état pathologique particulier ;
b) la quantification d'une fréquence d'interaction entre un segment d'acide nucléique et un segment d'acide nucléique cible ;
c) la comparaison de la fréquence d'interaction dans la composition d'acide nucléique provenant de l'individu avec ledit état pathologique avec la fréquence d'interaction dans une composition de noyaux témoin normale provenant d'un sujet en bonne santé, de sorte qu'une différence de la fréquence d'interaction dans la composition d'acide nucléique est indicative d'un état pathologique particulier.

9. Procédé selon la revendication 8, dans lequel l'état pathologique est sélectionné parmi un cancer, une maladie autoimmune, une maladie du développement ou un trouble génétique.

10. Utilisation d'un kit pour identifier des fragments d'acide nucléique qui interagissent avec un sous-groupe de segments d'acide nucléique cibles, qui comprend des tampons et des réactifs capables de réaliser le procédé selon l'une quelconque des revendications 1 à 7.


### SEQUENCE LISTING

<110> Babraham Institute
<120> CHROMOSOME CONFORMATION CAPTURE METHOD INCLUDING SELECTION AND ENRICHMENT STEPS
<130> BAB-C-P1604PCT
<160> 18
<170> PatentIn version 3.5
<210> 1
   <211> 32
   <212> DNA
   <213> Artificial
<220>
   <223> Synthetic Oligonucleotide
<400> 1
   gatcggaaga gcggttcagc aggaatgccg ag 32
<210> 2
   <211> 33
   <212> DNA
   <213> Artificial
<220>
   <223> Synthetic Oligonucleotide
<400> 2
   acactctttc cctacacgac gctcttccga tct 33
<210> 3
   <211> 58
   <212> DNA
   <213> Artificial
<220>
   <223> Synthetic Oligonucleotide
<400> 3
   aatgatacgg cgaccaccga gatctacact ctttccctac acgacgctct tccgatct 58
<210> 4
   <211> 58
   <212> DNA
   <213> Artificial
<220>
   <223> Synthetic Oligonucleotide
<400> 4
   caagcagaag acggcatacg agatcggtct cggcattcct gctgaaccgc tcttccga 58
<210> 5
   <211> 63
   <212> DNA
   <213> Artificial
<220>
   <223> Synthetic Oligonucleotide
<220>
   <221> misc_feature
   <222> (34)..(39)
   <223> n is a, c, g, or t
<400> 5
   gatcggaaga gcacacgtct gaactccagt cacnnnnnna tctcgtatgc cgtcttctgc 60
   ttg 63
<210> 6
   <211> 44
   <212> DNA
   <213> Artificial
<220>
   <223> Synthetic Oligonucleotide
<400> 6
   aatgatacgg cgaccaccga gatctacact ctttccctac acga 44
<210> 7
   <211> 24
   <212> DNA
   <213> Artificial
<220>
   <223> Synthetic Oligonucleotide
<400> 7
   caagcagaag acggcatacg agat 24
<210> 8
   <211> 33
   <212> DNA
   <213> Artificial
<220>
   <223> Synthetic Oligonucleotide
<400> 8
   gatcggaaga gcacacgtct gaactccagt cac 33
<210> 9
   <211> 33
   <212> DNA
   <213> Artificial
<220>
   <223> Synthetic Oligonucleotide
<400> 9
   gtgactggag ttcagacgtg tgctcttccg atc 33
<210> 10
   <211> 46
   <212> DNA
   <213> Artificial
<220>
   <223> Synthetic Oligonucleotide
<400> 10
   tctagtcgac ggccagtgaa ttgtaatacg actcactata gggcga 46
<210> 11
   <211> 50
   <212> DNA
   <213> Artificial
<220>
   <223> Synthetic Oligonucleotide
<400> 11
   agcttcgccc tatagtgagt cgtattacaa ttcactggcc gtcgactaga 50
<210> 12
   <211> 63
   <212> DNA
   <213> Artificial
<220>
   <223> Synthetic Oligonucleotide
<220>
   <221> misc_feature
   <222> (25)..(30)
   <223> n is a, c, g, or t
<400> 12
   caagcagaag acggcatacg agatnnnnnn gtgactggag ttcagacgtg tgctcttccg 60
   atc 63
<210> 13
   <211> 63
   <212> DNA
   <213> Artificial
<220>
   <223> Synthetic Oligonucleotide
<400> 13
   gatcggaaga gcacacgtct gaactccagt cacgccaata tctcgtatgc cgtcttctgc 60
   ttg 63
<210> 14
   <211> 63
   <212> **DNA**
   <213> Artificial
<220>
   <223> Synthetic Oligonucleotide
<400> **14**
   caagcagaag acggcatacg agatattggc gtgactggag ttcagacgtg tgctcttccg 60
   atc 63
<210> 15
   <211> 33
   <212> DNA
   <213> Artificial
<220>
   <223> Synthetic Oligonucleotide
<400> 15
   gtgactggag ttcagacgtg tgctcttccg atc 33
<210> 16
   <211> 33
   <212> DNA
   <213> Artificial
<220>
   <223> Synthetic Oligonucleotide
<400> 16
   agatcggaag agcgtcgtgt agggaaagag tgt 33
<210> 17
   <211> 34
   <212> DNA
   <213> Artificial
<220>
   <223> Synthetic Oligonucleotide
<400> 17
   agatcggaag agcacacgtc tgaactccag tcac 34
<210> 18
   <211> 32
   <212> DNA
   <213> Artificial
<220>
   <223> Synthetic Oligonucleotide
<400> 18
   acactctttc cctacacgac gctcttccga tc 32